# EUROPEAN PATENT APPLICATION

(11) **EP 1 875 912 A1**
(43) Date of publication of application: **09.01.2008**
(21) Application number: 06731927.7
(22) Date of filing: 10.04.2006
(51) Int. Cl.: A61K 31/444, A61K 31/506, A61P 25/14

(54) **AGENT FOR TREATING INVOLUNTARY MOVEMENT**

(30) Priority: 08.04.2005 JP 2005112137
(71) Applicant: Eisai R&D Management Co., Ltd., Tokyo 112-8088 (JP)
(72) Inventor: MIZUTA, Eiji, Meguro-ku Tokyo 152-8621 (JP); KUNO, Sadako, Meguro-ku Tokyo 152-8621 (JP); HANADA, Takahisa, Tsukuba-shi Ibaraki 300-2635 (JP); UENO, Masataka, Tsukuba-shi Ibaraki 300-2635 (JP)
(74) Representative: Woods, Geoffrey Corlett
(86) International application number: PCT/JP2006/307991
(87) International publication number: WO 2006/109876

(57) **Abstract**

The present invention relates to a therapeutic agent for dyskinesia excluding tremor, comprising 1,2-dihydropyridine compound, a salt thereof, or a solvate thereof, which shows AMPA receptor antagonism and is highly useful as a pharmaceutical drug.

## Description

### TECHNICAL FIELD

The present invention relates to a useful therapeutic agent for dyskinesia comprising a 1,2-dihydropyridine compound, a salt thereof, or a solvate thereof, which has AMPA receptor antagonism.

### BACKGROUND ART

Dyskinesia is involuntary or uncontrollable abnormality of physical motility, and includes, for example, tremor, chorea, ballismus, dystonia, athetosis, myoclonus, tic, and the like. Among these symptoms, most symptoms of tremor react well to therapeutic agents for Parkinson's disease, β-blockers and the like, and thus it is suggested that tremor decreases activity of dopaminergic nerve.

Dyskinesia is known to appear following neurodegenerative diseases, metabolic diseases or immune diseases, or following progress of these diseases. Examples of neurodegenerative diseases which are known to cause dyskinesia include cerebral ischemia, head injury, cerebral vascular disorder, Tourette syndrome, Parkinson's disease, Huntington chorea, spinocerebellar ataxia (atrophy of dentoliva, pons and cerebellum, dentatorubral-pallidoluysian atrophy, etc.), AIDS-related neuropathy, epilepsia, and neurodegeneration observed after hypoxia. Examples of metabolic diseases which are known to cause dyskinesia include acanthocytosis, Wilson's disease, glutaric acidemia, and Leigh disease. Examples of immune diseases which are known to cause dyskinesia include systemic lupus erythematosus, Sydenham's chorea, and chorea gravidarum. These diseases are critical diseases with many factors which have not been clarified including the mechanism of occurrence. No pharmaceutical agents useful for the therapy of these diseases have been found yet. Regarding dyskinesia which occurs following these diseases, no effective treating means has been found and thus such means is desired to be developed.

Dyskinesia is known to be induced by various drugs used for the therapy of various diseases. For example, it is known that dyskinesia is caused by administration of psychotropic agents (e.g., schizophrenia therapeutic agents, selective serotonin uptake inhibitors, tricyclic antidepressant, lithium, anti-epilepsy drugs), therapeutic agents for Parkinson's disease, cocaine, theophylline, ethanol, β adrenaline agonists, and the like. For example, for the therapy of psychiatric diseases, drugs having dopamine D2 (D₂) receptor antagonism are used, but psychotropic agents are known to cause abnormal movement mainly in the periphery of mouth when administered over a long period of time.

Drug-induced dyskinesia described above are known to be caused also by administration of antiemetic agents, agents for adjusting digestive system functions, agents for improving cerebral blood flow and metabolism, antihypertensives, and the like, although not as frequently as by the psychotropic agents.

As another example of the drug-induced dyskinesia, a new motility abnormality is known to be caused when a dopamine receptor agonist or a dopamine metabolism inhibitor is administered to a patient of Parkinson's disease or Parkinson's syndrome over a long period of time ^{(1,2)} Parkinson's disease or Parkinson's syndrome is known to be caused by degeneration of dopamine neurons of substantia nigra striate body. For the therapy of these symptoms, drugs such as dopamine receptor agonists, for example, bromocryptin, lisuride, pergolide, cabergoline, ropinirole, pramipexole, and L-DOPA; and dopamine metabolism inhibitors, for example, monoamine oxidase (MAO) inhibitor, catechol-O-methyl transferase (COMT) inhibitor are clinically applied or clinically used.

In general, for the therapy for dyskinesia excluding tremor, dopamine receptor antagonists represented by schizophrenia therapeutic agents or monoamine depletors represented by reserpine and tetrabenazine are used⁽³⁻⁶⁾. Based on this, it is presumed that abnormally increased activity of dopaminergic nerve exists behind the appearance of dyskinesia excluding tremor. However, these therapeutic agents need to be carefully used because they have side effects of provoking Parkinson's syndrome, excessive sedation and the like. Thus, a novel therapeutic method which does not cause much of these side effects is desired to be developed.

It has been reported that for the therapy of dyskinesia induced by administration of a dopamine receptor agonist or administration of a dopamine metabolism inhibitor, amantadine, which is a therapeutic agent for Parkinson's disease, is effective. However, it has also been reported that amantadine has a possibility of decreasing the effect of the other therapeutic agents for Parkinson's disease. Thus, it is considered that amantadine needs to be used with care⁽⁷⁾.

It has also been reported an N-methyl-D-asparaginic acid (NMDA)-type glutamic acid receptor antagonist exhibits an effect of improving the symptoms of dyskinesia⁽⁸⁾. However, many NMDA-type receptor antagonists are known to cause critical side effects on the human psychiatric system⁽⁹⁾.

It has been reported that an α-amino-3-hydroxy-5-methyl-4-isoxazole propionic acid (AMPA) receptor antagonist has an effect of enhancing the action of therapeutic agents for Parkinson's diseases. Namely, it has been reported that an AMPA receptor antagonist is usable as an adjuvant in the dopamine therapy of Parkinson's disease. However, the symptoms which can be improved by the AMPA receptor antagonist are limited to bradykinesia, tremor and muscle rigidity among various symptoms of Parkinson's diseases. Regarding the effect on dyskinesia based on abnormal activity of dopaminergic nerve, for example, chorea, dystonia, tic, ballismus, athetosis, and myoclonus, and the like, no knowledge has been shown^{(10-11,20)}.

It has been reported that a change in the reactivity of a Parkinson's disease patient to the therapeutic agent L-DOPA is involved in a change of the expression amount of the glutamic acid receptor and the like ⁽¹³⁾. It has been actually reported that the AMPA-type glutamate receptor antagonist can exhibit an effect of recovering the time period in which a dopamine receptor agonist is effective, which is shortened due to the long-time administration of L-DOPA. However, this action of the AMPA receptor antagonist of recovering the time period in which the dopamine receptor agonist is effective does not indicate that dyskinesia caused by the dopamine receptor agonist is suppressed, but merely indicates that the effect of the dopamine receptor agonist is enhanced^{(14,15)}_{.}

As understood from the above-mentioned reports, it was not necessarily recognized that an AMPA receptor antagonist is usable for the therapy of dyskinesia. However, recently, the following studies have been made regarding the application of an AMPA receptor antagonist for the therapy of dyskinesia. When monkeys selected as experimental animals, which have been treated with MPTP (1-methyl-4-phenyl-1,2,3,6-tetrahydropyridine) to cause selective degeneration of substantia nigra melamine-containing neuron, are administered with L-DOPA as a dopamine receptor agonist for 4 to 5 weeks, dyskinesia newly occurs in correspondence with an anti-Parkinsonian effect of L-DOPA after dose. When LY-300164 (Talampanel) known as an AMPA receptor antagonist was administered to the monkeys in such a state, the dyskinesia were improved⁽¹⁶⁾. With rhesus monkey models, it has been indicated that an AMPA receptor antagonist having a quinazoline structure has an effect on dyskinesia⁽²¹⁾. Also with rhesus monkey models, it has been reported that 3-(2-chlorophenyl)-2-[2-(6-diethylaminomethylpyridine-2-yl)-vinyl]-6-fluoro-3H-quinazoline-4-on has an effect on dyskinesia⁽²²⁾. With marmoset models, it has been reported that Topiramate, as an anti-epilepsy drug which is considered to have AMPA receptor antagonism, has an action of suppressing dyskinesia ⁽¹⁷⁾. An AMPA receptor antagonist has also been reported to have an effect on dystonia⁽¹⁸⁾. It has also been reported that in a patient of tardive dyskinesia, a marker of excitatory neurotransmission caused by glutamic acid is raised in cerebrospinal fluid⁽¹⁹⁾. From the above-mentioned reports, an AMPA receptor antagonist is expected to have a therapeutic effect on naturally occurring dyskinesia, and dyskinesia based on abnormal activity of dopaminergic nerve which is caused by administration of drugs such as dopamine receptor agonists.

As described above, for the therapy of dyskinesia excluding tremor, it is required to avoid side effects of provoking Parkinson's syndrome, excessive sedation and the like, which are caused by the conventional therapeutic agents. However, the study reports on the AMPA receptor antagonist as a therapeutic agent for dyskinesia do not describe or suggest that the AMPA receptor antagonist clinically alleviates the symptoms of Parkinson's syndrome caused as side effects. Namely, in the experiment with the monkeys, LY-300164 is not recognized as having an effect of clearly improving the symptoms of Parkinson's disease, i.e., as having an effect of ameliorating the symptom scores⁽¹⁶⁾. The AMPA receptor antagonist having a quinazoline structure⁽²¹⁾ and 3-(2-chlorophenyl)-2-[2-(6-diethylaminomethylpyridine-2-yl)-vinyl]-6-fluoro-3H-quina zoline-4-on⁽²²⁾ are described as having an effect on dyskinesia, but are not described as having an effect on the symptoms of Parkinson's disease, which are side effects thereof. In addition, Topiramate shows a tendency of deteriorating the symptoms of Parkinson's disease, although not significantly, and thus has a similar problem as the conventional therapeutic agents⁽¹⁷⁾. NBQX, which expresses nephrotoxicity, has been given up for the use on the humans⁽¹⁸⁾.

As compounds having AMPA receptor antagonism, competitive AMPA receptor antagonist compounds having a quinoxalinedion structure^{(23.25)}, non-competitive AMPA receptor antagonist compounds(^{26.33}), and the like have been reported.

1,2-dihydropyridine compounds have conventionally been reported as being used as a ligand of GABA_{A} receptor α subunit⁽³⁴⁾, for the therapy of epilepsy⁽³⁵⁾, for the therapy of various nerve diseases⁽³⁶⁾, and the like.

As described above, there are many reports on an AMPA receptor antagonist, and some studies- have been made on the use thereof for the therapy of dyskinesia. However, any compound, which has a therapeutic effect on dyskinesia and does not exhibit the above-mentioned side effects involved in the therapy of dyskinesia excluding tremor, has not been found yet. Thus, a compound effectively acting clinically without any side effect described above as means for treating dyskinesia excluding tremor is desired to be created.

### Reference documents

(1) Jenner, P., Neurology, 2002, 58 (Suppl 1): S1-8
(2) Jankovic, J., Neurology, 2002, 58(Suppl 1): S19-32
(3) O'Brien CF. Chorea. In: Jankovic J and Tolosa E eds. Parkinson's Disease and Movement Disorders. Baltimore: Lippincot Williams & Wilkins. 1998, pp. 357-364
(4) Shannon KM. Ballism. In: Jankovic J and Tolosa E eds. Parkinson's Disease and Movement Disorders. Baltimore: Lippincot Williams & Wilkins. 1998, pp. 365-375
(5) Tolosa E and Jankovic J. Tics and Tourette's Syndrome. In: Jankovic J and Tolosa E eds. Parkinson's Disease and Movement Disorders. Baltimore: Lippincot Williams & Wilkins. 1998, 491-512
(6) Brin MF. Treatment of Dystonia. In: Jankovic J and Tolosa E eds. Parkinson's Disease and Movement Disorders. Baltimore: Lippincot Williams & Wilkins. 1998, pp. 553-578
(7) P.J.Blanchet Mov Disord. 1998; 13(5): 798-802
(8) S.M.Papa Ann Neurol 1996; 39: 574-578
(9) Olney et al., J Psychiatr Res 1999; 33: 523-533
(10) P.A.Loschmann et al., J Neural Trnasm 1991; 3: 203-213
(11) J Neural Trnasm 1992; Suppl 38: 55-64
(12) T.Klockgether et al., Ann Neurol 1991; 30: 717-723
(13) Calom, F.et al., Neurobiol Dis 2003, 14; 404-416
(14) C.Marin et al., Synapse. 2000 15; 36(4): 267-74
(15) Synapse. 2001; 42(1): 40-7
(16) T.N.Chase et al., Neurology 2000; 54: 1589-1595
(17) A.Monty et al., Mov Disord. 2005, 20(4); 403-409
(18) Richter A.et al., Eur. J. Pharmacol. 1993, 231; 287-291
(19) G.Tsai et al., Am J Psychiatry. 1998, 155; 1207-1213
(20) USP 6, 191,132 specification
(21) USP 6,136,812 specification
(22) US Laid-Open Patent Publication No. 2001/0034345 specification
(23) International Publication No. 94/25469 pamphlet
(24) International Publication No. 96/10023 pamphlet
(25) USP 5,356,902 specification
(26) International Publication No. 95/01357 pamphlet
(27) International Publication No. 97/28135 pamphlet
(28) International Publication No. 97/28163 pamphlet
(29) International Publication No. 97/43276 pamphlet
(30) International Publication No. 97/34878 pamphlet
(31) International Publication No. 98/38173 pamphlet
(32) European Patent No. 802195 specification
(33) USP 6,277,872 specification
(34) International Publication No. 98/55480 pamphlet
(35) International Publication No. 00/07988 pamphlet
(36) International Publication No. 01/96308 pamphlet

### DISCLOSURE OF THE INVENTION

The present invention is for providing a useful therapeutic agent for dyskinesia (excluding tremor) which exhibits excellent AMPA receptor antagonism and has novel features not provided conventionally

The present inventors built a hypothesis that a 1,2-dihydropyridine compound having excellent AMPA receptor antagonism can be a good antidyskinesic agent and conducted active studies.

As a result, the present inventors found that a 1,2-dihydropyridine compound, preferably 3-(2-cyanophenyl)-5-(2-pyridyl)-1 -phenyl-1,2-dihydropyridine-2-on (International Publication No. 01/96308 pamphlet, Example 7) unexpectedly has features, not found in the conventional compounds, of providing an dyskinesia suppressing action without causing side effects, especially without causing the symptoms of Parkinson's disease, which would otherwise be caused as a result of activity of dopaminergic nerve being suppressed and was considered a problem in the conventional therapy of dyskinesia excluding tremor. The present inventors clarified that such a compound, owing to these novel features, can effectively act as an active component of an dyskinesia therapeutic agent useful for treating dyskinesia excluding tremor, preferably, as an active component of an dyskinesia therapeutic agent based on abnormal activity of dopaminergic nerve, and thus completed the present invention.

The present invention is as follows.
(1) A therapeutic agent for dyskinesia (excluding tremor), comprising a compound represented by the following general formula (1), a salt thereof, or a solvate thereof: 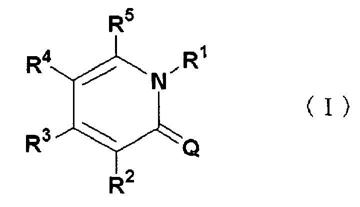 (in the formula,
   Q represents =NH, =O or =S;
   R¹, R², R³, R⁴ and R⁵ identically or differently represent a group represented by a hydrogen atom, a halogen atom, a C₁-C₆ alkyl group or a group represented by formula -X-A;
   X represents a single bond, a C₁-C₆ alkylene group which may have a substituent, a C₂-C₆ alkenylene group which may have a substituent, a C₂-C₆ alkynylene group which may have a substituent, -O-, -S-, -CO-, -SO-, -SO₂-, -N(R⁶)-, -N(R⁷)-CO-, -CO-N(R⁸)-, -N(R⁹)-CH₂-, -CH₂-N(R¹⁰)-, -CH₂-CO-, -CO-CH₂-, -N(R¹¹)-S(O)ₘ, -S(O)ₙ-N(R¹²)-, -CH₂-S(O)p-, -S(O)q-CH₂-, -CH₂-O-, -O-CH₂-, -N(R¹³)-CO-N(R¹⁴)-, or -N(R¹⁵)-CS-N(R¹⁶)-;
   R⁶, R⁷ R⁸, R⁹, R¹⁰, R¹¹, R¹², R¹³, R¹⁴ R¹⁵ and R¹⁶ identically or differently represent a hydrogen atom, a C₁-C₆ alkyl group or a C₁-C₆ alkoxy group;
   m, n, p and q independently represent an integer 0, 1 or 2; and
   A represents a C₃-C₈ cycloalkyl group, a C₃-C₈ cycloalkenyl group, a 5- to 14-membered non-aromatic heterocyclic group, a C₆-C₁₄ aromatic hydrocarbon cyclic group, or a 5- to 14-membered aromatic heterocyclic group, each of which may have a substituent;
   wherein three groups among R¹, R², R³, R⁴ and R⁵ always identically or differently represent a group represented by -X-A, and the remaining two groups always represent a hydrogen atom, a halogen atom or a C₁-C₆ alkyl group).
(2) The therapeutic agent according to (1), wherein the dyskinesia is dyskinesia based on abnormally increased activity of dopaminergic nerve.
(3) The therapeutic agent according to (1), wherein the dyskinesia is at least one selected from the group consisting of chorea, dystonia, tic, ballismus, athetosis, and myoclonus.
(4) The therapeutic agent according to (1), wherein the dyskinesia is at least one selected from the group consisting of dyskinesia (excluding tremor) which occurs following neurodegenerative diseases, metabolic diseases or immune diseases, and a drug-induced dyskinesia (excluding tremor).
(5) The therapeutic agent according to (4), wherein the dyskinesia (excluding tremor) which occurs following neurodegenerative diseases is dyskinesia (excluding tremor) which occurs following at least one selected from the group consisting of Tourette syndrome, spinocerebellar ataxia, cerebral vascular disorder, and head injury.
(6) The therapeutic agent according to (4), wherein the dyskinesia (excluding tremor) which occurs following metabolic diseases is dyskinesia (excluding tremor) which occurs following at least one selected from the group consisting of acanthocytosis, Wilson's disease, glutaric academia, and Leigh disease.
(7) The therapeutic agent according to (4), wherein the dyskinesia (excluding tremor) which occurs following immune diseases is dyskinesia (excluding tremor) which occurs following at least one selected from the group consisting of systemic lupus erythematosus, Sydenham's chorea, and chorea gravidarum.
(8) The therapeutic agent according to (4), wherein the drug-induced dyskinesia (excluding tremor) is dyskinesia (excluding tremor) which occurs following administration of a psychotropic agent and/or a dopamine receptor agonist.
(9) The therapeutic agent according to (4), wherein the drug-induced dyskinesia (excluding tremor) is dyskinesia (excluding tremor) which occurs following administration of a dopamine receptor agonist.
(10) The therapeutic agent according to (4), wherein the drug-induced dyskinesia (excluding tremor) is dyskinesia (excluding tremor) which occurs following combined use of L-DOPA or a prodrug thereof and a peripheral dopadecarboxylase inhibitor.
(11) The therapeutic agent according to (1), wherein the compound is at least one selected from the group consisting of 3-(2-cyanophenyl)-5-(2-pyridyl)-1-phenyl-1,2-dihydropyridine-2-on, 3-(2-cyanophenyl)-5-(2-pyridyl)-1-(3-pyridyl)-1,2-dihydropyridine-2-on, 3-(2-fluoropyridine-3-yl)-5-(2-pyridyl)-1-phenyl-1,2-dihydropyridine-2-on, 3-(2-fluoropyridine-3-yl)-5-(2-pyridyl)-1-(3-pyridyl)-1,2-dihydropyridine-2-on, 3-(2-cyanophenyl)-1-phenyl-5-(2-pyrimidinyl)-1,2-dihydropyridine-2-on, 3-(2-cyanophenyl)-1-(3-pyridyl)-5-(2-pyrimidinyl)-1,2-dihydropyridine-2-on, 3-(2- fluoropyridine-3-yl)-1-phenyl-5-(2-pyrimidinyl)-1,2-dihydropyridine-2-on, and 3-(2-cyanopyridine-3-yl)-1-phenyl-5-(2-pyrimidinyl)-1,2-dihydropyridine-2-on.
(12) The therapeutic agent according to (1), wherein the compound is 3-(2-cyanophenyl)-5-(2-pyridyl)-1-phenyl-1, 2-dihydropyridine-2-on.
(13) The therapeutic agent according to (1), wherein the compound is a hydrate 3-(2-cyanophenyl)-5-(2-pyridyl)-1-phenyl-1,2-dihydropyridine-2-on hydrate.

The present invention provides an excellent therapeutic agent for dyskinesia (excluding tremor). More specifically, an excellent therapeutic agent for dyskinesia (excluding tremor) comprising a 1,2-dihydropyridine compound, i.e., a compound represented by general formula (I), preferably 3-(2-cyanophenyl)-5-(2-pyridyl)-1-phenyl-1,2-dihydropyridine-2-on, a salt thereof, or a solvate thereof is provided, and is made usable for the therapy of dyskinesia excluding tremor.

The present invention makes it possible to perform an dyskinesia therapy without causing the symptoms of Parkinson's disease, which would otherwise be caused as a result of activity of dopaminergic nerve being suppressed and was considered a problem in the therapy of dyskinesia excluding tremor, and preferably while clearly improving the symptoms of Parkinson's disease.

### BRIEF DESCRIPTION OF THE DRAWINGS

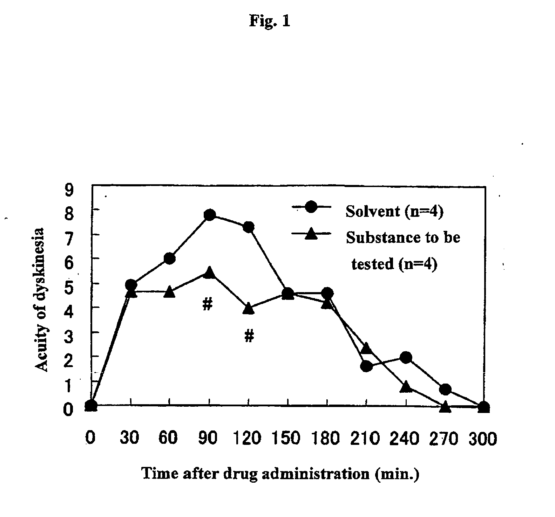

Fig. 1 shows the effects of the substance tested on dyskinesia. The vertical axis represents the severity of dyskinesia, and the horizontal axis represents the time after L-DOPA methylester and benserazide are administered.
   "#" indicates that there is a statistically significant difference with respect to the solvent (P < 0.01).

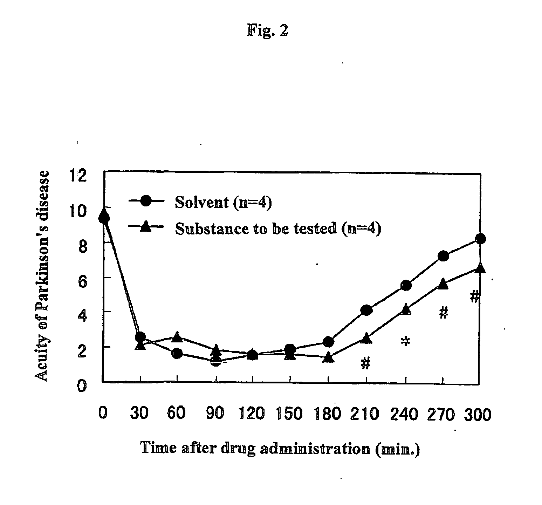

Fig. 2 shows the effects of the substance tested on the symptoms of Parkinson's disease. The vertical axis represents the severity of dyskinesia, and the horizontal axis represents the time after L-DOPA methylester and benserazide are administered. "*" and "#" each indicate that there is a statistically significant difference with respect to the solvent (P < 0.05, P < 0.01, respectively).

### BEST MODE FOR CARRYING OUT THE INVENTION

Hereinafter, embodiments of the present invention will be described. The following embodiments are given in order to illustrate the present invention and are not intended to limit the present invention in any way. The present invention can be carried out in various embodiments without departing from the scope thereof.

The prior art documents, laid-open publications, patents and other patent documents cited in this specification are all incorporated herein by reference.

### 1. 1,2-dihydropyridine compound

A therapeutic agent according to the present invention contains a 1,2-dihydropyridine compound.

According to the present invention, the 1,2-dihydropyridine compound encompasses a compound represented by the following general formula (I), a salt thereof, and a solvate thereof.

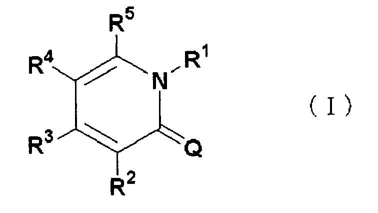

In general formula (I), Q represents =NH, =O or =S, and is preferably =O.

In general formula (I), R¹, R², R³, R⁴ and R⁵ identically or differently represent a hydrogen atom, a halogen atom, a C₁-C₆ alkyl group or a group represented by a formula -X-A.

Herein, examples of the "halogen atom" include atoms such fluorine atom, chlorine atom, bromine atom, iodine atom, and the like.

Herein, the "C₁-C₆ alkyl group" represents a straight-chain or branched-chain alkyl group having a carbon number of 1 to 6. Examples of such a group include methyl group, ethyl group, n-propyl group, isopropyl group, n-butyl group, isobutyl group, sec-butyl group (1-methylpropyl group), tert-butyl group, n-pentyl group, isopentyl group, tert-pentyl group (1,1-dimethylpropyl group), 1,2-dimethylpropyl group, 2,2-dimethylpropyl group (neopentyl group), 1-ethylpropyl group, 1-methylbutyl group, 2-methylbutyl group, n-hexyl group, isohexyl group, 1-ethyl-1-methylpropyl group, 1-ethyl-2-methylpropyl group, 1,1,2-trimethylpropyl group, 1,2,2-trimethylpropyl group, 1,2-dimethylbutyl group, 1,1-dimethylbutyl group, 2,2-dimethylbutyl group, 1,3-dimethylbutyl group, 2,3-dimethylbutyl group, 3,3-dimethylbutyl group, 1-ethylbutyl group, 2-ethylbutyl group, 1-methylpentyl group, 2-methylpentyl group, 3-methylpentyl group, and the like.

Herein, the group expressed by "X" in the formula -X-A represents:
a single bond,
a C₁-C₆ alkylene group which may have a substituent,
a C₂-C₆ alkenylene group which may have a substituent,
a C₂-C₆ alkynylene group which may have a substituent,
-O-, -S-, -CO-, -SO-, -SO₂-, -N(R⁶)-, -N(R⁷)-CO-, -CO-N(R⁸)-, -N(R⁹)-CH₂-, -CH₂-N(R¹⁰)-, -CH₂-CO-, -CO-CH₂-, -N(R¹¹)-S(O)ₘ-, -S(O)ₙ-N(R 12)-, -CH₂-S(O)p-, -S(O)q-CH₂-, -CH₂-O-, -O-CH₂-, -N(R¹³)-CO-N(R¹⁴)-, or -N(R¹⁵)-CS-N(R¹⁶)-_{;}
R⁶, R⁷, R⁸, R⁹, R¹⁰, R¹¹, R¹², R¹³, R¹⁴, R¹⁵ and R¹⁶ identically or differently represent a hydrogen atom, a C₁-C₆ alkyl group or a C₁-C₆ alkoxy group; or
m, n, p and q independently represent an integer 0, 1 or 2.

Herein, the expression "may have a substituent" means that "may have one or a plurality of substituents in an optional combination at a substitutable site".

Herein, the "C₁-C₆ alkylene group" represents an alkylene group having a carbon number of 1 to 6. The "C₁-C₆ alkylene group" especially preferable as X is an alkylene group having a carbon number of 1 to 3, and examples of such a "C₁-C₆ alkylene group" include -CH₂-, -(CH₂)₂-, -CH(CH₃)-, -C(CH₃)₂-, -(CH₂)₃-, -CH(CH₃)-CH₂-, -CH₂-CH(CH₃)-, and the like.

Herein, the "C₂-C₆ alkenylene group" represents an alkenylene group having a carbon number of 2 to 6. The C₂-C₆ alkenylene group especially preferable as X is an alkenylene group having a carbon number of 2 or 3, and examples of such an alkenylene group include -CH=CH-, -CH=CH-CH₂-, -CH₂-CH=CH-, -C(CH₃)=CH-, -CH=C(CH₃)-, and the like.

Herein, the "C₂-C₆ alkynylene group" represents an alkynylene group having a carbon number of 2 to 6. The C₂-C₆ alkynylene group especially preferable as X is an alkynylene group having a carbon number of 2 or 3, and examples of such an alkynylene group include -C≡C-, -C≡C-CH₂-, -CH₂-C≡C-, and the like.

Preferable examples of the "substituent" in the "C₁-C₆ alkylene group which may have a substituent", "C₂-C₆ alkenylene group which may have a substituent", and "C₂-C₆ alkynylene group which may have a substituent" represented by X include halogen atom (e.g., fluorine atom, chlorine atom, bromine atom, iodine atom, etc.), hydroxyl group, nitrile group, nitro group, and the like.

Herein, the "C₁-C₆ alkoxy group" represents an alkoxy group having a carbon number of 1 to 6, which corresponds to the C₁-C₆ alkyl group above. Examples of "C₁-C₆ alkoxy group" as used herein include methoxy group, ethoxy group, n-propoxy group, isopropoxy group, n-butoxy group, isobutoxy group, sec-butoxy group, tert-butoxy group, n-pentyloxy group, isopentyloxy group, tert-pentyloxy group, 1,2-dimethylpropoxy group, neopentyloxy group, 1-ethylpropoxy group, 1-methylbutoxy group, 2-methylbutoxy group, n-hexyloxy group, isohexyloxy group, 1-ethyl-1-methylpropoxy group, 1-ethyl-2-methylpropoxy group, 1,1,2-trimethylpropoxy group, 1,2,2-trimethylpropoxy group, 1,2-dimethylbutoxy group, 1,1-dimethylbutoxy group, 2,2-dimethylbutoxy group, 1,3-dimethylbutoxy group, 2,3-dimethylbutoxy group, 3,3-dimethylbutoxy group, 1-ethylbutoxy group, 2-ethylbutoxy group, 1-methylpentyloxy group, 2-methylpentyloxy group, 3-methylpentyloxy group, and the like.

Preferable examples of the "C₁-C₆ alkyl group" in the group represented by R⁶ R⁷, R⁸, R⁹, R¹⁰, R¹¹, R¹² R¹³, R¹⁴, R¹⁵ and R¹⁶ include methyl group, ethyl group, n-propyl group, isopropyl group, n-butyl group, tert-butyl group, and the like. Preferable examples of the "C₁-C₆ alkoxy group" in the group represented by R⁶, R⁷, R⁸, R⁹, R¹⁰, R¹¹, R¹², R¹³, R¹⁴, R¹⁵ and R¹⁶ include methoxy group, ethoxy group, n-propoxy group, isopropoxy group, n-butoxy group, tert-butoxy group, and the like.

Herein, preferable examples of the group represented by X include a single bond, -CH₂-, -CH(OH)-, -CH(CN)-, -CH₂-CH₂-, -CH(OH)-CH₂-, -CH(CN)-CH₂-, -CH₂-CH(OH)-, -CH₂-CH(CN)-, -CH=CH-, -CH=CH-CH₂-, -CH=CH-CH(OH)-, -CH=CH-CH(CN)-, -CH(OH)-CH=CH-, -CH(CN)-CH=CH-, -C=C-, -O-, -S-, -SO-, -SO₂-, -CO-, -NH-CO-NH-, -NH-CS-NH-, and the like. Among these, a single bond, -CH₂-; -CH(OH)-, -CH(CN)-, -CH₂-CH₂-, -CH(OH)-CH₂-, -CH(CN)-CH₂-, -CH₂-CH(OH)-, -CH₂-CH(CN)-, -CH=CH-, -C≡C-, -CO-, and the like are more preferable. A single bond, -CH₂-, -CH(OH)-, and -CO- are still more preferable, and a single bond is most preferable.

Herein, the group expressed by "A" in the formula -X-A represents:
a C₃-C₈ cycloalkyl group which may have a substituent,
a C₃-C₈ cycloalkenyl group which may have a substituent,
a 5- to 14-membered non-aromatic heterocyclic group which may have a substituent,
a C₆-C₁₄ aromatic hydrocarbon cyclic group which may have a substituent, or
a 5- to 14-membered aromatic heterocyclic group which may have a substituent.

Herein, the "C₃-C₈ cycloalkyl group" represents a cycloalkyl group formed of 3 to 8 carbon atoms, and examples of such a group include cyclopropyl group, cyclobutyl group, cyclopentyl group, cyclohexyl group, cycloheptyl group, cyclooctyl group, and the like.

Herein, the "C₃-C₈ cycloalkenyl group" represents a cycloalkenyl group formed of 3 to 8 carbon atoms, and examples of such a group include cyclopropene-1-yl, 2-cyclopropene-1-yl, cyclobutene-1-yl, 2-cyclobutene-1-yl, 1,3-cyclobutadiene-1-yl, cyclopentene-1-yl, 2-cyclopentene-1-yl, 3-cyclopentene-1-yl, 1,3-cyclopentadiene-1-yl, 1,4-cyclopentadiene-1-yl, 2,4-cyclopentadiene-1-yl, cyclohexene-1-yl, 2-cyclohexene-1-yl, 3-cyclo hexene-1-yl, 1, 3-cyclohexadiene-1-yl, 1,4-cyclohexadiene-1-yl, 1,5-cyclohexadiene-1-yl, 2,4-cyclohexadiene-1-yl, 2,5-cyclohexadiene-l-yl, cycloheptene-1-yl, 2-cycloheptene-1-yl, 3-cycloheptene-1-yl, 4-cycloheptene-1-yl, 1,3-cycloheptadiene-1-yl, 1,4-cycloheptadiene-1-yl, 1,5-cycloheptadiene-1-yl, 1,6-cycloheptadiene-1-yl, 2,4-cycloheptadiene-1-yl, 2,5-cycloheptadiene-1-yl, 2,6-cycloheptadiene-1-yl, 3,5-cycloheptadiene-1-yl, 1,3,5-cycloheptatolyene-1-yl, 1,3,6-cycloheptatolyene-1-yl, 1,4,6-cycloheptatolyene-1-yl, 2,4,6-cycloheptatolyene-1-yl, cyclooctene-1-yl, 2-cyclooctene-1-yl, 3-cyclooctene-1-yl, 4-cyclooctene-1-yl, 1,3-cyclooctadiene-1-yl, 1,4-cyclooctadiene-1-yl, 1,5-cyclooctadiene-1-yl, 1,6-cyclooctadiene-1-yl, 1,7-cyclooctadiene-1-yl, 2,4-cyclooctadiene-1-yl, 2,5-cyclooctadiene-1-yl, 2,6-cyclooctadiene-1-yl, 2,7-cyclooctadiene-1-yl, 3,5-cyclooctadiene-1-yl, 3,6-cyclooctadiene-1-yl, 1,3,5-cyclooctatolyene-1-yl, 1,3,6-cyclooctatolyene-1-yl, 1,3,7-cyclooctatolyene-1-yl, 1,4,6-cyclooctatolyene-1-yl, 1,4,7-cyclooctatolyene-1-yl, 1,5,7-cyclooctatolyene-1-yl, 2,4,6-cyclooctatolyene-1-yl, 2,4,7-cyclooctatolyene-1-yl group, and the like.

Herein, the "5- to 14-membered non-aromatic heterocyclic group" refers to a monocyclic, bicyclic, tricyclic or other polycyclic 5- to 14-membered non-aromatic heterocyclic group containing one or more heteroatoms selected from the group consisting of nitrogen atom, sulfur atom and oxygen atom. Specific examples of such a group include pyrrolidinyl group, pyrrolinyl group, piperidyl group, piperazinyl group, piperidinyl group, imidazolidinyl group, pyrazolidinyl group, morpholinyl group, tetrahydrofuryl group, tetrahydropyranyl group, dihydrofuryl group, dihydropyranyl group, imidazolinyl group, oxazolinyl group, and the like. Such a non-aromatic heterocyclic group encompasses a group derived from pyridone ring and a non-aromatic fused ring (e.g., a group derived from a phthalimide ring, a succinimide ring or the like).

Herein, each of the "C₆-C₁₄ aromatic hydrocarbon cyclic group" and the "aryl group" refers to an aromatic hydrocarbon cyclic group formed of 6 to 14 carbon atoms, and encompasses a monocyclic, bicyclic, tricyclic or other polycyclic group and a fused ring thereof. Specific examples of such a group include phenyl group, indenyl group, 1-naphthyl group, 2-naphthyl group, azulenyl group, heptalenyl group, biphenyl group, indacenyl group, acenaphthyl group, fluorenyl group, phenalenyl group, phenanthrenyl group, anthracenyl group, cyclopentacyclooctenyl group, benzocyclooctenyl group, and the like.

Herein, each of the "5- to 14-membered aromatic heterocyclic group" and the "heteroaryl group" refers to a 5- to 14-membered aromatic heterocyclic group containing one or more heteroatoms selected from the group consisting of nitrogen atom, sulfur atom and oxygen atom, and encompasses a monocyclic, bicyclic, tricyclic or other polycyclic group and a fused ring thereof. Specific examples of such a group include:
1) as nitrogen-containing aromatic heterocyclic groups, pyrrolyl group, pyridyl group, pyridazinyl group, pyrimidinyl group, pyrazinyl group, triazolyl group, tetrazolyl group, benzotriazolyl group, pyrazolyl group, imidazolyl group, benzimidazolyl group, indolyl group, isoindolyl group, indolizinyl group, purinyl group, indazolyl group, quinolyl group, isoquinolyl group, quinolizyl group, phthalazyl group, naphthylidinyl group, quinoxalyl group, quinazolinyl group, cinnolinyl group, pteridinyl group, imidazotriazinyl group, pyrazinopyridazinyl group, acrydinyl group, phenanthridinyl group, carbazolyl group, carbolinyl group, perimidinyl group, phenanthrolinyl group, phenazinyl group, imidazopyridyl group, imidazopyrimidinyl group, pyrazolopyridyl group, and the like;
2) as sulfur-containing aromatic heterocyclic groups, thienyl group, benzothienyl group, and the like;
3) as oxygen-containing aromatic heterocyclic groups, furyl group, pyranyl group, cyclopentapyranyl group, benzofuryl group, isobenzofuryl group, dioxinyl group, and the like, and
4) as aromatic heterocyclic groups containing two or more different types of heteroatoms, thiazolyl group, isothiazolyl group, benzothiazolyl group, benzothiadiazolyl group, phenothiazinyl group, isoxazolyl group, furazanyl group, phenoxazinyl group, oxazolyl group, benzoxazolyl group, oxadiazolyl group, pyrazolooxazolyl group, imidazothiazolyl group, thienofuranyl group, phlopyrrolyl group, pyridoxadinyl group, and the like.

There is no specific limitation on preferable examples of the group represented by "A", but more preferable examples of such a group include phenyl group, pyrrolyl group, pyridyl group, pyridazinyl group, pyrimidinyl group, pyrazinyl group, thienyl group, thiazolyl group, furyl group, naphthyl group (1-naphthyl group, 2-naphthyl group), quinolyl group, isoquinolyl group, indolyl group, benzimidazolyl group, benzothiazolyl group, benzoxazolyl group, imidazopyridyl group, carbazolyl group, cyclopentyl group, cyclohexyl group, cyclohexenyl group (cyclohexene-1-yl group, 2-cyclohexene-1-yl group, 3-cyclohexene-1-yl group), dioxinyl group, pyrrolidinyl group, piperidyl group, piperazinyl group, morpholinyl group, and the like, each of which may have a substituent.

Still more preferable examples of such a group are, for example, expressed by the formula: 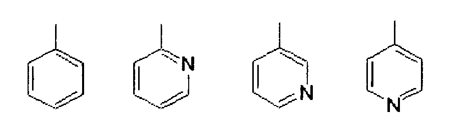 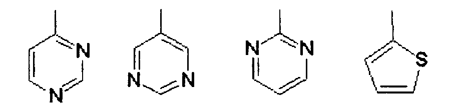 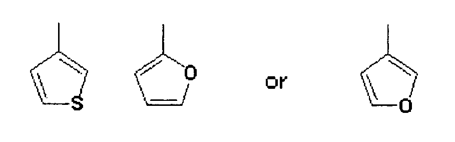 each of which may have a substituent. Most preferable examples of such a group are, for example, expressed by the formula: 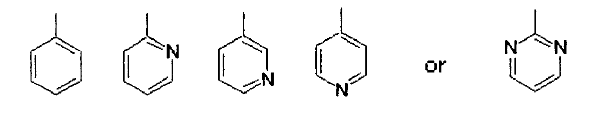 each of which may have a substituent.

Herein, preferable examples of the "substituent" in the group represented by "A" include:
groups such as hydroxyl group, halogen atom, nitrile group, nitro group, and the like;
C₁-C₆ alkyl group, C₂-C₆ alkenyl group, and C₂-C₆ alkynyl group, each of which may have a substituent;
C₁-C₆ alkoxy group, C₂-C₆ alkenyloxy group, and C₂-C₆ alkynyloxy group, each of which may have a substituent;
C₁-C₆ alkylthio group, C₂-C₆ alkenylthio group, and C₂-C₆ alkynylthio group, each of which may have a substituent;
amino group which may have a substituent;
substituted carbonyl group;
C₁-C₆ alkylsulfonyl group, C₂-C₆ alkenylsulfonyl group, C₂-C₆ alkynylsulfonyl group, C₁-C₆ alkylsulfinyl group, C₂-C₆ alkenylsulfinyl group, and C₂-C₆ alkynylsulfinyl group, each of which may have a substituent;
formyl group;
aralkyl group, heteroarylalkyl group, aralkyloxy group, and heteroarylalkyloxy group, each of which may have a substituent;
C₃-C₈ cycloalkyl group, and C₃-C₈ cycloalkenyl group, each of which may have a substituent; and
5- to 14-membered non-aromatic heterocyclic group, C₆-C₁₄ aromatic hydrocarbon cyclic group, and 5- to 14-membered aromatic heterocyclic group, each of which may have a substituent.

Herein, the "C₂-C₆ alkenyl group" refers to an alkenyl group having a carbon number of 2 to 6. Preferable example of such a group include vinyl group, 1-ethylethenyl group (1-butene-2-yl group), allyl group (2-propenyl group), 1-propenyl group, isopropenyl group, 2-methyl-1-propenyl group, 1-methyl-1-propenyl group (2-butene-2-yl group), 2-methyl-2-propenyl group, 1-methyl-2-propenyl group (l-butene-3-yl group), 1-butenyl group (1-butene-l-yl group), 2-butenyl group (2-butene-l-yl group), 3-butenyl group, 1-pentenyl group, 1-hexenyl group, 1,3-hexanedienyl group, 1,6-hexanedienyl group, isopentenyl group, and-the like.

Herein, the "C₂-C₆ alkynyl group" refers to an alkynyl group having a carbon number of 2 to 6. Preferable examples of such a group include ethynyl group, 1-propynyl group, 2-propynyl group, 1-butynyl group, 2-butynyl group, 3-butynyl group, 1-methyl-2-propynyl group, 1-ethyl-2-propynyl group, 1-ethynyl-2-propynyl group, 2-methyl-3-butenyl group, 1-pentynyl group, 1-hexynyl group, 1,3-hexanediinyl group, 1,6-hexanediinyl group, and the like.

Herein, the "C₂-C₆ alkenyloxy group" refers to an alkenyloxy group having a carbon number of 2 to 6. Preferable examples of such a group include vinyloxy group, 1-ethylethenyloxy group (1-butene-2-yloxy group), allyloxy group (2-propenyloxy group), 1-propenyloxy group, isopropenyloxy group, 2-methyl-1-propenyloxy group, 1-methyl-1-propenyloxy group (2-butene-2-yloxy group), 2-methyl-2-propenyloxy group, 1-methyl-2-propenyloxy group (1-butene-3-yloxy group), 1-butenyloxy group (1-butene-1-yloxy group), 2-butenyloxy group (2-butene-1-yloxy group), 3-butenyloxy group, 1-pentenyloxy group, isopentenyloxy group, 1-hexenyloxy group, 1,3-hexanedienyloxy group, 1,6-hexanedienyloxy group, and the like.

Herein, the "C₂-C₆ alkynyloxy group" refers to an alkynyloxy group having a carbon number of 2 to 6. Preferable examples of such a group include ethynyloxy group, 1-propynyloxy group, 2-propynyloxy group, 1-butynyloxy group, 2-butynyloxy group, 3-butynyloxy group, 1-methyl-2-propynyloxy group, 1-ethyl-2-propynyloxy group, 1-ethynyl-2-propynyloxy group, 1-pentynyloxy group, 1-hexynyloxy group, 1,3-hexanediinyloxy group, 1,6-hexanediinyloxy group, and the like.

Preferable examples of the "halogen atom" listed above as the "substituent" of the group represented by "A" include fluorine atom, chlorine atom, bromine atom, iodine atom, and the like. Fluorine atom, chlorine atom, and bromine atom are more preferable.

Preferable examples of the "C₁-C₆ alkyl group which may have a substituent" listed above as the "substituent" of the group represented by "A" include methyl group, ethyl group, n-propyl group, isopropyl group, n-butyl group, isobutyl group, 1-methylpropyl group, tert-butyl group, n-pentyl group, isopentyl group, 1,2-dimethylpropyl group, neopentyl group, 1-methylbutyl group, 2-methylbutyl group, n-hexyl group, 1-ethyl-2-methylpropyl group, 1,1,2-trimethylpropyl group, 1,2-dimethylbutyl group, 1,1-dimethylbutyl group, 2,2-dimethylbutyl group, 1,3-dimethylbutyl group, 2-ethylbutyl group, 2-methylpentyl group, 3-methylpentyl group, and the like, each of which may have a substituent.

Preferable examples of the "C₂-C₆ alkenyl group which may have a substituent" listed above as the "substituent" of the group represented by "A" include vinyl group, allyl group, 1-propenyl group, isopropenyl group, 1-butene-1-yl group, 1-butene-2-yl group, 1-butene-3-yl group, 2-butene-1-yl group, 2-butene-2-yl group, isopentenyl group and the like, each of which may have a substituent.

Preferable examples of the "C₂-C₆ alkynyl group which may have a substituent" listed above as the "substituent" of the group represented by "A" include ethynyl group, 1-propynyl group, 2-propynyl group, butynyl group, pentynyl group, hexynyl group, and the like, each of which may have a substituent.

The "substituent" in each of the "C₁-C₆ alkyl group which may have a substituent", "C₂-C₆ alkenyl group which may have a substituent", and "C₂-C₆ alkynyl group which may have a substituent" listed above as the "substituent" of the group represented by "A" may be, for example, one or more groups selected from the "group of substituents A".

### <Group of substituents A>

hydroxyl group, nitrile group, halogen atom, N-(C₁-C₆) alkylamino group, N,N-di(C₁-C₆) alkylamino group, N-(C₂-C₆) alkenylamino group, N,N-di(C₂-C₆) alkenylamino group, N-(C₂-C₆) alkynylamino group, N,N-di(C₂-C₆) alkynylamino group, C₆-C₁₄ aromatic hydrocarbon cyclic group (e.g., phenyl group, etc.), 5- to 14-membered aromatic heterocyclic group (e.g., thienyl group, furyl group, pyridyl group, pyridazinyl group, pyrimidinyl group, pyrazinyl group, etc.), aralkyloxy group, heteroaryloxy group, TBDMS oxy group, C₁-C₆ alkylsulfonylamino group, C₂-C₆ alkenylsulfonylamino group, C₂-C₆ alkynylsulfonylamino group, C₁-C₆ alkylcarbonyloxy group, C₂-C₆ alkenylcarbonyloxy group, C₂-C₆ alkynylcarbonyloxy group, C₁-C₆ alkylcarbamoyl group, C₂-C₆ alkenylcarbamoyl group, C₂-C₆ akynylcarbamoyl group, and the like.

Preferable examples of the "C₁-C₆ alkoxy group which may have a substituent" listed above as the "substituent" of the group represented by "A" include methoxy group, ethoxy group, n-propoxy group, isopropoxy group, sec-propoxy group, n-butoxy group, isobutoxy group, sec-butoxy group, tert-butoxy group, n-pentyloxy group, isopentyloxy group, tert-pentyloxy group, 1,2-dimethylpropoxy group, neopentyloxy group, 2-methylbutoxy group, n-hexyloxy group, isohexyloxy group, 1-ethyl-2-methylpropoxy group, 1,1,2-trimethylpropoxy group, 1,2,2-trimethylpropoxy group, 1,2-dimethylbutoxy group, 1,1-dimethylbutoxy group, 2,2-dimethylbutoxy group, 1,3-dimethylbutoxy group, 2-ethylbutoxy group, 2-methylpentyloxy group, 3-methylpentyloxy group, and the like, each of which may have a substituent.

Preferable examples of the "C₂-C₆ alkenyloxy group which may have a substituent" listed above as the "substituent" of the group represented by "A" include vinyloxy group, allyloxy group, 1-propenyloxy group, isopropenyloxy group, 1-butene-1-yloxy group, 1-butene-2-yloxy group, 1-butene-3-yloxy group, 2-butene-1-yloxy group, 2-butene-2-yloxy group, each of which may have a substituent.

Preferable examples of the "C₂-C₆ alkynyloxy group which may have a substituent" listed above as the "substituent" of the group represented by "A" include ethynyloxy group, 1-propynyloxy group, 2-propynyloxy group, butynyloxy group, pentynyloxy group, hexynyloxy group, and the like, each of which may have a substituent.

The "substituent" in each of the "C₁-C₆ alkoxy group which may have a substituent", "C₂-C₆ alkenyloxy group which may have a substituent", and "C₂-C₆ alkynyloxy group which may have a substituent" may be, as a preferable example, one or more groups selected from C₁-C₆ alkylamino group, aralkyloxy group, hydroxyl group and the like.

Preferable examples of each of the "C₁-C₆ alkylthio group which may have a substituent", "C₂-C₆ alkenylthio group which may have a substituent", and "C₂-C₆ alkynylthio group which may have a substituent" listed above as the "substituent" of the group represented by "A" include C₁-C₆ alkylthio group (e.g., methylthio group, ethylthio group, n-propylthio group, isopropylthio group, n-butylthio group, isobutylthio group, tert-butylthio group, n-pentylthio group, isopentylthio group, neopentylthio group, n-hexylthio group, etc.), C₂-C₆ alkenylthio group (e.g., vinylthio group, allylthio group, 1-propenylthio group, isopropenylthio group, 1-butene-1-ylthio group, 1-butene-2-ylthio group, 1-butene-3-ylthio group, 2-butene-1-ylthio group, 2-butene-2-ylthio group, etc.), and, C₂-C₆ alkynylthio group (e.g., ethynylthio group, 1-propynylthio group, 2-propynylthio group, butynylthio group, pentynylthio group, hexynylthio group, etc.), each of which may be substituted with one or more groups selected from the group consisting of hydroxyl group, halogen atom, nitrile group and nitro group.

The "substituent" of the "amino group which may have a substituent" listed above as the "substituent" of the group represented by "A" may be, as an example, one or two groups selected from C₁-C₆ alkyl group, C₂-C₆ alkenyl group, C₂-C₆ alkynyl group, C₁-C₆ alkylsulfonyl group, C₂-C₆ akenylsulfonyl group, C₂-C₆ alkynylsulfonyl group, C₁-C₆ alkylcarbonyl group, C₂-C₆ alkenylcarbonyl group, C₂-C₆ alkynylcarbonyl group, and the like, each of which may have a substituent. Preferable examples of the "substituent" in the C₁-C₆ alkyl group, C₂-C₆ alkenyl group, C₂-C₆ alkynyl group, C₁-C₆ alkylsulfonyl group, C₂-C₆ alkenylsulfonyl group, C₂-C₆ alkynylsulfonyl group, C₁-C₆ alkylcarbonyl group, C₂-C₆ alkenylcarbonyl group and C₂-C₆ alkynylcarbonyl group include hydroxyl group, halogen atom, nitrile group, C₁-C₆ alkoxy group, C₁-C₆ alkylthio group, and the like.

Especially preferable examples of the "amino group which may have a substituent" specifically include:
methylamino group, ethylamino group, n-propylamino group, isopropylamino group, n-butylamino group, isobutylamino group, 1-methylpropylamino group, tert-butylamino group, n-pentylamino group, isopentylamino group, 1,2-dimethylpropylamino group, neopentylamino group, 1-methylbutylamino group, 2-methylbutylarriino group, n-hexylamino group, 1-ethyl-2-methylpropylamino group, 1,1,2-trimethylpropylamino group, 1,2-dimethylbutylamino group, 1,1-dimethylbutylamino group, 2,2-dimethylbutylamino group, 1,3-dimethylbutylamino group, 2-ethylbutylamino group, 2-methylpentylamino group, 3-methylpentylamino group, N,N-dimethylamino group, N,N-diethylamino group, N,N-di(n-propyl)amino group, N,N-di(isopropyl)amino group, N,N-di(n-butyl)amino group, N,N-di(isobutyl)amino group, N,N-di(tert-butyl)amino group, N,N-di(n-pentyl)amino group, N,N-di(isopentyl)amino group, N,N-di(neopentyl)amino group, N,N-di(n-hexyl)amino group, N,N-di(1-methylpropyl)amino group, N,N-di(1,2-dimethylpropyl)amino group, N-methyl-N-ethylamino group, N-ethyl-N-(n-propyl)amino group, N-methyl-N-(isopropyl)amino group,
vinylamino group, allylamino group, (1-propenyl)amino group, isopropenylamino group, (1-butene-1-yl)amino group, (1-butene-2-yl)amino group, (1-butene-3-yl)amino group, (2-butene-1-yl)amino group, (2-butene-2-yl)amino group, N,N-divinylamino group, N,N-diallylamino group, N,N-di(1-propenyl)amino group, N,N-diisopropenylamino group, N-vinyl-N-allylamino group, ethynylamino group, 1-propynylamino group, 2-propynylamino group, butynylamino group, pentynylamino group, hexynylamino group, N,N-diethynylamino group, N,N-(1-propynyl)amino group, N,N-(2-propynyl)amino group, N,N-dibutynylamino group, N,N-dipentynylamino group, N,N-dihexynylamino group,
hydroxymethylamino group, 1-hydroxyethylamino group, 2-hydroxyethylamino group, 3-hydroxy-n-propylamino group, methylsulfonylamino group, ethylsulfonylamino group, n-propylsulfonylamino group, isopropylsulfonylamino group, n-butylsulfonylamino group, tert-butylsulfonylamino group, vinylsulfonylamino group, allylsulfonylamino group, isopropenylsulfonylamino group, isopentenylsulfonylamino group,
ethynylsulfonylamino group,
methylcarbonylamino group, ethylcarbonylamino group, n-propylcarbonylamino group, isopropylcarbonylamino group, n-butylcarbonylamino group, tert-butylcarbonylamino group,
vinylcarbonylamino group, allylcarbonylamino group, isopropenylcarbonylamino group, isopentenylcarbonylamino group,
ethynylcarbonylamino group, and the like.

Preferable examples of the "substituted carbonyl group" listed above as the "substitute" of the group represented by "A" include groups represented by formula -CO-W (examples of W in the formula include C₁-C₆ alkyl group, C₂-C₆ alkenyl group, C₂-C₆ alkynyl group, C₁-C₆ alkoxy group, amino group, N-(C₁-C₆) alkylamino group, N,N-di(C₁-C₆ alkyl)amino group, N-(C₂-C₆) alkenylamino group, N,N-di(C₂-C₆ alkenyl)amino group, N-(C₂-C₆) alkynylamino group, N,N-di(C₂-C₆ alkynyl)amino group, N-(C₁-C₆) alkyl-N-(C₂-C₆) alkenylamino group, N-(C₁-C₆) alkyl-N-(C₂-C₆) alkynylamino group, N-(C₂-C₆) alkenyl-N-(C₂-C₆) alkynylamino group, and the like).

Preferable examples of each of the "C₁-C₆ alkylsulfonyl group which may have a substituent", "C₂-C₆ alkenylsulfonyl group which may have a substituent", "C₂-C₆ alkynylsulfonyl group which may have a substituent", "C₁-C₆ alkylsulfinyl group which may have a substituent", "C₂-C₆ alkenylsulfinyl group which may have a substituent", and "C₂-C₆ alkynylsulfinyl group which may have a substituent" listed above as the "substitute" of the group represented by "A" include:
methylsulfonyl group, ethylsulfonyl group, n-propylsulfonyl group, isopropylsulfonyl group, n-butylsulfonyl group, tert-butylsulfonyl group,
vinylsulfonyl group, allylsulfonyl group, isopropenylsulfonyl group, isopentenylsulfonyl group,
ethynylsulfonyl group,
methylsulfinyl group, ethylsulfinyl group, n-propylsulfinyl group, isopropylsulfinyl group, n-butylsulfinyl group, tert-butylsulfinyl group,
vinylsulfinyl group, allylsulfinyl group, isopropenylsulfinyl group, isopentenylsulfinyl group,
ethynylsulfinyl group, and the like,
each of which may have a substituent.

The C₁-C₆ alkylsulfonyl group, C₂-C₆ alkenylsulfonyl group, C₂-C₆ alkynylsulfonyl group, C₁-C₆ alkylsulfinyl group, C₂-C₆ alkenylsulfinyl group and C₂-C₆ alkynylsulfinyl group may each have, as a substituent, one or more selected from the above-mentioned group of substituents A.

Preferable examples of each of the "aralkyl group which may have a substituent" and "heteroarylalkyl group which may have a substituent" listed above as the "substituent" of the group represented by A include benzyl group, phenetyl group, naphthylmethyl group, naphthylethyl group, pyridylmethyl group, pyridylethyl group, thienylmethyl group, thienylethyl group, and the like, each of which may have a substituent.

Preferable examples of the "aralkyloxy group which may have a substituent" listed above as the "substituent" of the group represented by A include benzyloxy group, phenetyloxy group, phenylpropyloxy group, naphthylmethyloxy group, naphthylethyloxy group, naphthylpropyloxy group, and the like, each of which may have a substituent.

Preferable examples of the "heteroarylalkyloxy group which may have a substituent" listed above as the "substituent" of the group represented by A include pyridylmethyloxy group, pyrazinylmethyloxy group, pyrimidinylmethyloxy group, pyrrolylmethyloxy group, imidazolylmethyloxy group, pyrazolylmethyloxy group, quinolylmethyloxy group, isoquinolylmethyloxy group, furfuryloxy group, thienylmethyloxy group, thiazolylmethyloxy group, and the like, each of which may have a substituent.

The aralkyl group, heteroarylalkyl group, aralkyloxy group, and heteroarylalkyloxy group may each have, as a substituent, one or more selected from the above-mentioned group of substituents A.

Preferable examples of each of the "C₃-C₈ cycloalkyl group which may have a substituent" and "C₃-C₈ cycloalkenyl group which may have a substituent" listed above as the "substituent" of the group represented by A include:
cyclopropyl group, cyclobutyl group, cyclopentyl group, cyclohexyl group, cycloheptanyl group, and the like; each of which may have a substituent; and
cyclopropenyl group, cyclobutenyl group, cyclopentenyl group, cyclohexenyl group, cycloheptenyl group, and the like, each of which may have a substituent. These groups may be each substituted with one or more groups selected from the group of substituents B.

### <Group of substituents B>

hydroxyl group, halogen atom, nitrile group, C₁-C₆ alkyl group (e.g., methyl group, ethyl group, n-propyl group, isopropyl group, n-butyl group, isobutyl group, tert-butyl group, n-pentyl group, isopentyl group, neopentyl group, n-hexyl group, etc.), C₁-C₆ alkoxy group (e.g., methoxy group, ethoxy group, n-propoxy group, isopropoxy group, sec-propoxy group, n-butoxy group, isobutoxy group, sec-butoxy group, tert-butoxy group, n-pentoxy group, isopentoxy group, sec-pentoxy group, tert-pentoxy group, n-hexoxy group, etc.), C₁-C₆ alkoxy C₁-C₆ alkyl group (e.g., methoxymethyl group, methoxyethyl group, ethoxymethyl group, ethoxyethyl group, etc.), aralkyl group (e.g., benzyl group, phenetyl group, naphthylmethyl group, naphthylethyl group, etc.), and the like.

Regarding the "5- to 14-membered non-aromatic heterocyclic group which may have a substituent", "C₆-C₁₄ aromatic hydrocarbon cyclic group which may have a substituent", and "5- to 14-membered aromatic heterocyclic group which may have a substituent" each listed above as the "substitute" of the group represented by A, there is no specific limitation on "5- to 14-membered non-aromatic heterocyclic group", "C₆-C₁₄ aromatic hydrocarbon cyclic group" and "5- to 14-membered aromatic heterocyclic group".

More preferable examples of the "5- to 14-membered non-aromatic heterocyclic group" include pyrrolidinyl group, pyrrolinyl group, piperidyl group, piperazinyl group, imidazolinyl group, pyrazolidinyl group, imidazolidinyl group, morpholinyl group, phthalimidyl group, succinimidyl group, and the like, each of which may have a substituent.

More preferable examples of the "C₆-C₁₄ aromatic hydrocarbon cyclic group" include phenyl group, indenyl group, naphthyl group, azulenyl group, heptalenyl group, biphenyl group, and the like, each of which may have a substituent.

More preferable examples of the "5- to 14-membered aromatic heterocyclic group" include pyrrolyl group, pyridyl group, pyridazinyl group, pyrimidinyl group, pyrazinyl group, pyrazolyl group, imidazolyl group, thienyl group, furyl group, thiazolyl group, isothiazolyl group, quinolyl group, isoquinolyl group, indolyl group, benzimidazolyl group, benzothiazolyl group, benzoxazolyl group, imidazopyridyl group, carbazolyl group, dioxinyl group, and the like, each of which may have a substituent. The "substituent" in the context of "may have a substituent" may be, as a preferable example, one or more groups selected from the above-mentioned group of substituents B. Amino group, cyclic amino group, and alkoxyamino group, each of which may have a substituent, are preferable as such a substituent.

In general formula (I), three groups among R¹, R², R³, R⁴ and R⁵ always identically or differently represent a group represented by -X-A, and the remaining two groups always represent a hydrogen atom, a halogen atom or a C₁-C₆ alkyl group.

In the present invention, there is no specific limitation on preferable embodiments of the 1,2-dihydropyridine compound, namely, a compound represented by general formula (1), a salt thereof, or a solvate thereof: 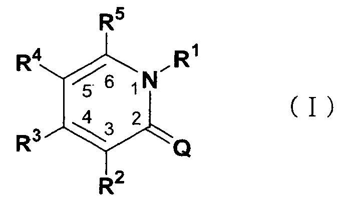 (in the formula, Q, R¹, R², R³, R⁴ and R⁵ each have the same meaning as defined above). For example, an embodiment of the 1,2-dihydropyridine compound according to the present invention is a compound in which:
R¹ is a group represented by the formula -X-A (X and A each have the same meaning as defined above), and
two of the remaining R², R³, R⁴ and R⁵ are each a group represented by the formula -X-A (X and A each have the same meaning as defined above), and the other two are each a hydrogen atom, a halogen atom or a C₁-C₆ alkyl group, a salt thereof, or a solvate thereof; i.e., a compound represented by formula (II): 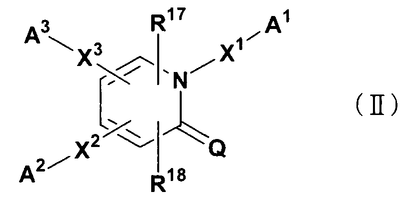 (in the formula, Q has the same meaning as in general formula (I); X¹, X² and X³ independently have the same meaning as that of X in general formula (I); A¹, A² and A³ independently have the same meaning as that of A in general formula (I); and R¹⁷ and R¹⁸ identically or differently represent a hydrogen atom, a halogen atom or a C₁-C₆ alkyl group), a salt thereof, or a solvate thereof.

A more preferable embodiment of the 1,2-dihydropyridine compound according to the present invention is a compound in which Q in formula (II) is an oxygen atom, and R¹⁷ and R¹⁸ in formula (II) are position 4 and position 6 of a pyridone ring, a salt thereof, or a solvate thereof; namely, a pyridone compound represented by general formula (III): 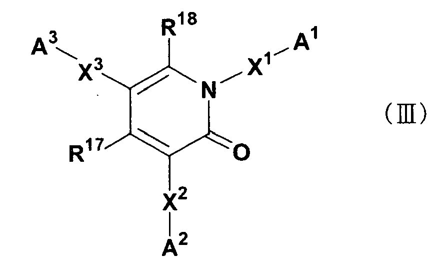 (in the formula, X¹, X², X³, A¹, A², A³, R¹⁷ and R¹⁸ each have the same meaning as defined above), a salt thereof, or a solvate thereof.

A still more preferable embodiment of the 1,2-dihydropyridine compound according to the present invention is a compound in which R¹⁷ and R¹⁸ in formula (III) are each a hydrogen atom, namely, a 1,3,5-substituted pyridone compound represented by formula (IV): 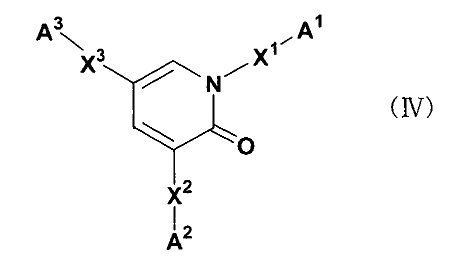 (in the formula, X¹, X², X³, A¹, A² and A³ each have the same meaning as defined above), a salt thereof, or a solvate thereof.

A most preferable embodiment of the 1,2-dihydropyridine compound according to the present invention is a compound in which X¹, X² and X³ in formula (IV) are each a single bond, namely, a 1,3,5-substituted pyridone compound represented by formula (V): 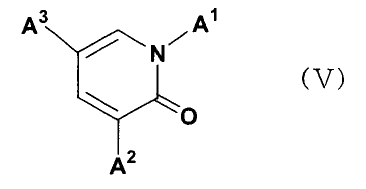 (in the formula, A¹, A² and A³ each have the same meaning as defined above), a salt thereof, or a solvate thereof. Preferable examples of A¹, A² and A³ are as listed above regarding A.

Preferable examples of the compound represented by general formula (I) according to the present invention include the following compounds.
3-(2-cyanophenyl)-5-(2-pyridyl)-1-phenyl-1,2-dihydropyridine-2-on 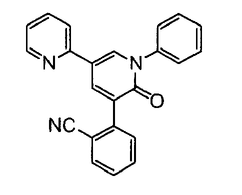 3-(2-cyanophenyl)-5-(2-pyridyl)-1-(3-pyridyl)-1,2-dihydropyridine-2-on 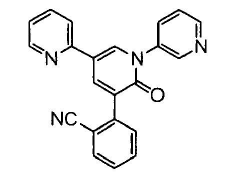 3-(2-fluoropyridine-3-yl)-5-(2-pyridyl)-1-phenyl-1,2-dihydropyridine-2-on 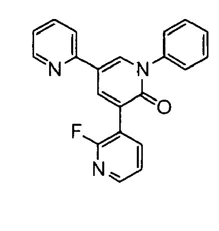 3-(2-fluoropyrid ine-3-yl)-5-(2-pyridyl)-1-(3-pyridyl)-1,2-dihydropyridine-2-on 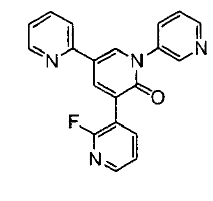 3-(2-cyanophenyl)-1-phenyl-5-(2-pyrimidinyl)-1,2-dihydropyrid'me-2-on 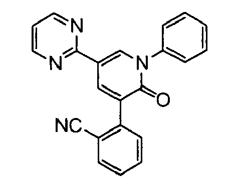 3-(2-cyanophenyl)-1-(3-pyridyl)-5-(2-pyrimidinyl)-1,2-dihydropyridine-2-on 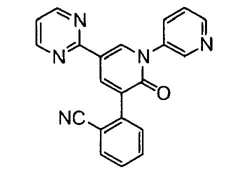 3-(2-fluoropyridine-3-yl)-1-phenyl-5-(2-pyrimidinyl)-1,2-dihydropyridine-2-on 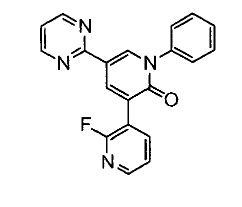 3-(2-cyanopyridine-3-yl)-1-phenyl-5-(2-pyrimidinyl)-1,2-dihydropyridine-2-on

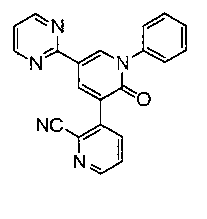

According to the present invention, a more preferable example of the compound represented by general formula (I) is 3-(2-cyanophenyl)-5-(2-pyridyl)-1-phenyl-1,2-dihydropyridine-2-on (see International Publication No.01/96308 pamphlet, Example 7). 3-(2-cyanophenyl)-5-(2-pyridyl)-1-phenyl-1,2-dihydropyridine-2-on is preferably a hydrate.

Herein, a structural formula of a compound may represent a certain isomer for the sake of convenience, but the present invention encompasses all the isomers and isomer mixtures including geometric isomers generated by the structure of the compound, optical isomers based on asymmetric carbon, rotational isomers, stereoisomers, tautomers and the like. A compound according to the present invention is not limited to any structural formula provided for the sake of convenience, and may be an isomer or a mixture of them. Accordingly, the 1,2-dihydropyridine compound represented by general formula (I) of the present invention may have an asymmetric carbon atom in the molecule thereof and thus include an optical activator or a racemate. Such compounds are also encompassed in the present invention with no limitation. The 1,2-dihydropyridine compound represented by general formula (I) may also include polymorphism, and any of the morphisms may be a single body or a mixture of morphisms.

According to the present invention, the compound represented by general formula (I) or a salt thereof may be anhydride, or in the case where there is a solvate, may be a solvate thereof. All of these are encompassed in the scope of the 1,2-dihydropyridine compound according to the present invention. The solvate may be a hydrate or a non-hydrate, and preferably is a hydrate. Usable examples of the non-hydrate include alcohol (e.g., methanol, ethanol, n-propanol), dimethylformamide, and the like.

According to the present invention, the 1,2-dihydropyridine compound also encompasses a compound represented by general formula (I) which is metabolized *in vivo,* for example, oxidized, reduced, hydrolyzed, conjugated or the like, and a metabolite generated as a result of the *in vivo* metabolization. According to the present invention, the 1,2-dihydropyridine compound encompasses a compound (prodrug) for generating a compound, represented by general formula (I) (including a salt thereof, or a solvate thereof), as a result of the *in vivo* metabolization, for example, oxidation, reduction, hydrolysis, conjugation, or the like.

Herein, the term "salt" refers to any substance which forms a salt with the compound represented by general formula (I) and is pharmacologically acceptable, with no specific limitation. Preferable examples of such a salt include hydrohalic acid salt (e.g., hydrochloride, hydrobromide, hydroiodide, etc.), inorganic acid salt (e.g., sulfate, nitrate, perchlorate, phosphate, carbonate, bicarbonate, etc.), organic carboxylate (e.g., acetate, trifluoroacetate, maleate, tartrate, fumarate, citrate, etc.), organic sulfonate (e.g., methanesulfonate, trifluoromethanesulfonate, ethanesulfonate, benzenesulfonate, toluenesulfonate, camphorsulfonate, etc.), amino acid salt (e.g., aspartate, glutamate, etc.), quaternary amine salt, alkaline metal salt (e.g., sodium salt, potassium salt, etc.), alkaline-earth metal salt (magnesium salt, calcium salt, etc.). A more preferable "pharmacologically acceptable salt" according to the present invention is hydrochloride.

According to the present invention, the 1,2-dihydropyridine compound represented by general formula (I), a salt thereof, or a solvate thereof may be produced by a known method. The 1,2-dihydropyridine compound represented by general formula (I), a salt thereof, or a solvate thereof may be easily produced by, typically, a method disclosed in International Publication No.01/96308 pamphlet or a method conforming thereto. For example, the compound represented by general formula (I) of the present invention, any of 3-(2-cyanophenyl)-5-(2-pyridyl)-1-phenyl-1,2-dihydropyridine-2-on, 3-(2-cyanophenyl)-5-(2-pyridyl)-1-(3-pyridyl)-1,2-dihydropyridine-2-on, 3-(2-fluoropyridine-3-yl)-5-(2-pyridyl)-1 -phenyl-1,2-dihydropyridine-2-on, 3-(2-fluoropyridine-3-yl)-5-(2-pyridyl)-1-(3-pyridyl)-1,2-dihydropyridine-2-on, 3-(2-cyanophenyl)-1-phenyl-5-(2-pyrimidinyl)-1,2-dihydropyridine-2-on, 3-(2-cyanophenyl)-1 -(3-pyridyl)-5-(2-pyrimidinyl)-1,2-dihydropyridine-2-on, 3-(2-fluoropyridine-3-yl)-1-phenyl-5-(2-pyrimidinyl)-1,2-dihydropyridine-2-on, and 3-(2-cyanopyridine-3-yl)-I-phenyl-5-(2-pyrimidinyl)-1,2-dihydropyridine-2-on may be easily produced by a known method, typically, a method disclosed in International Publication No.01/96308 pamphlet or a method conforming thereto. 3-(2-cyanophenyl)-5-(2-pyridyl)-1-phenyl-1,2-dihydropyridine-2-on may be easily produced by a known method disclosed in International Publication No. 01/96308 pamphlet, Example 7 or a method conforming thereto.

### 2. Pharmaceutical composition

A pharmaceutical composition according to the present invention comprises a 1,2-dihydropyridine compound represented by general formula (I), a salt thereof, or a solvate thereof as an active component.

The 1,2-dihydropyridine compound, a salt thereof, or a solvate thereof is as described in section "1. 1,2-dihydropyridine compound".

The 1,2-dihydropyridine compound represented by general formula (I) which is contained in a pharmaceutical composition according to the present invention is preferably at least one compound selected from 3-(2-cyanophenyl)-5-(2-pyridyl)-1-phenyl-1,2-dihydropyridine-2-on, 3-(2-cyanophenyl)-5-(2-pyridyl)-1-(3-pyridyl)-1,2-dihydropyridine-2-on, 3-(2-fluoropyridine-3-yl)-5-(2-pyridyl)-1-phenyl-1,2-dihydropyridine-2-on, 3-(2-fluoropyridine-3-yl)-5-(2-pyridyl)-1-(3-pyridyl)-1,2-dihydropyridine-2-on, 3-(2-cyanophenyl)-1-phenyl-5-(2-pyrimidinyl)-1,2-dihydropyridine-2-on, 3-(2-cyanophenyl)-1-(3-pyridyl)-5-(2-pyrimidinyl)-1,2-dihydropyridine-2-on, 3-(2-fluoropyridine-3-yl)-1 -phenyl-5-(2-pyrimidinyl)-1,2-dihydropyridme-2-on, and 3-(2-cyanopyridine-3-yl)-1-phenyl-5-(2-pyrimidinyl)-1,2-dihydropyridine-2-on, a salt thereof, or a solvate thereof; is more preferably 3-(2-cyanophenyl)-5-(2-pyridyl)-1-phenyl-1,2-dihydropyridine-2-on, a salt thereof, or a solvate thereof; and still more preferably a hydrate of 3-(2-cyanophenyl)-5-(2-pyridyl)-1-phenyl-1,2-dihydropyridine-2-on.

The 1,2-dihydropyridine compound represented by general formula (I), a salt thereof, or a solvate thereof exhibits excellent AMPA receptor antagonism and is highly useful as a pharmaceutical composition (International Publication No. 01/96308 pamphlet). Therefore, the pharmaceutical composition according to the present invention is useful as a pharmaceutical composition, especially as a pharmaceutical composition to be used for the therapy of dyskinesia (excluding tremor).

According to the present invention, the compound represented by general formula (I), a salt thereof, or a solvate thereof may be produced into a formulation by a commonly used method. Examples of the form of the formulation include tablet, powdered drug, fine granule, granule, coated tablet, capsule, syrup, troche, inhalant, suppository, formulation for injection, ointment, ophthalmic ointment, eye drop, nose drop, ear drop, poultice, lotion and the like. For producing a formulation, a generally used excipient, binder, disintegrator, lubricant, coloring agent, flavor, and the like, and optionally, stabilizer, emulsifier, absorption enhancer, surfactant, pH adjuster, preservative, antioxidant and the like, are usable. A formulation can be produced by a usual method by mixing components which are generally used as materials of pharmaceutical preparations.

Usable components include, for example, (1) animal and vegetable oils including soybean oil, beef tallow, synthetic glyceride, and the like; (2) hydrocarbons including liquid paraffin, squalene, solid paraffin, and the like; (3) ester oils including octyldodecyl myristate, isopropyl myristate, and the like; (4) higher alcohols including cetostearylic alcohol, behenyl alcohol, and the like; (5) silicone resins; (6) silicone oils; (7) surfactants including polyoxyethylene fatty acid ester, sorbitan fatty acid ester, glycerin fatty acid ester, polyoxyethylenesorbitan fatty acid ester, polyoxyethylene cured castor oil, polyoxyethylene-polyoxypropylene block copolymer, and the like; (8) water soluble polymers including hydroxyethylcellulose, polyacrylic acid, carboxyvinyl polymer, polyethyleneglycol, polyvinylpyrrolidone, methylcellulose; and the like; (9) lower alcohols including ethanol, isopropanol and the like; (10) polyhydric alcohols including glycerin, propyleneglycol, dipropyleneglycol, sorbitol, and the like; (11) sugars including glucose, sucrose, and the like; (12) inorganic powders including silicic anhydride, magnesium aluminum silicate, aluminum silicate, and the like; (13) purified water; and the like.

The following components which are permitted to be added to pharmaceutical drugs are usable:
1) as the excipient, for example, lactose, cornstarch, white sugar, dextrose, mannitol, sorbite, crystalline cellulose, silicon dioxide, and the like;
2) as the binder, for example, polyvinyl alcohol, polyvinyl ether, methylcellulose, ethylcellulose, Arabic gum, tragacanth, gelatin, shellac, hydroxypropylcellulose, hydroxypropylmethylcellulose, polyvinylpyrrolidone, polypropyleneglycol-polyoxyethylene block polymer, meglumine, calcium citrate, dextrin, pectin and the like;
3) as the disintegrator, for example, starch, agar, powdered gelatin, crystalline cellulose, calcium carbonate, sodium hydrogen carbonate, calcium citrate, dextrin, pectin, carboxymethylcellulose-calcium, and the like;
4) as the lubricant, for example, magnesium stearate, talc, polyethyleneglycol, silica, cured vegetable oil, and the like;
5) as the coloring agent, any substance which is permitted to be added to pharmaceutical drugs;
6) as the flavor, powdered cocoa, menthol, aromatic, mint oil, borneol, powdered cinnamon bark, and the like; and
7) as the antioxidant, ascorbic acid, α-tocopherol, and the like.

An oral formulation may be produced as follows. To the 1,2-dihydropyridine compound, i.e., a compound represented by general formula (I), a salt thereof, or a solvate thereof, an excipient and optionally, a binder, a disintegrator, a lubricant, a coloring agent, a flavor or the like are added. Then the resultant substance is produced into powdered drug, fine granule, granule, tablet, coated tablet, capsule, troche, or the like using any conventional method.

The table or granule may be optionally coated with appropriate sugar, gelatin or the like.

A liquid formulation such as syrup, inhalant, formulation for injection, eye drop, nose drop, ear drop, lotion or the like may produced by a usual method by adding a pH adjuster, an isotonizing agent such as a resolvent, or the like and, optionally, a solubilizer, a stabilizer, a buffering agent, a suspending agent, an antioxidant or the like. A liquid formulation may be produced into a lyophilized substance, and a formulation for injection may be administered intravenously (including intravenous dripping), subcutaneously, or intramuscularly. Preferable examples of the suspending agent include methylcellulose, polysorbate 80, hydroxyethylcellulose, Arabic gum, tragacanth powder, carboxymethylcellulose sodium, polyoxyethylenesorbitan monolaurate, and the like. Preferable examples of the solubilizer include polyoxyethylene cured castor oil, polysorbate 80, amide nicotinate, polyoxyethylenesorbitan monolaurate, and the like. Preferable examples of the stabilizer include sodium sulfite, sodium metasulfite, ether, and the like. Preferable examples of the preservative include methyl paraoxybenzoate, ethyl paraoxybenzoate, sorbic acid, phenol, cresol, chlorocresol, and the like.

An external formulation such as suppository, ointment, ophthalmic ointment, poultice, or the like may be produced by a usual method with no specific limitation. As the base, any of various materials generally used for pharmaceutical drugs, quasi-pharmaceutical drugs, cosmetics and the like is usable. Examples of the base include animal and vegetable oils, mineral oils, ester oils, waxes, higher alcohols, fatty acids, silicone oils, surfactants, phospholipids, alcohols, polyhydric alcohols, water soluble polymers, clay minerals, purified water, and the like. Optionally, any of pH adjuster, antioxidant, chelating agent, preservative/fungicide, coloring agent, scenting agent and the like may be added. Also optionally, any of differentiation-inducing agent, blood flow promoter, disinfectant, anti-inflammatory, cell activator, vitamins, amino acid, humectant, keratolytic and the like may be mixed.

The dose of the pharmaceutical composition according to the present invention varies in accordance with the degree of symptoms, age, gender, body weight, form of administration, type of salt, sensitivity to the drug, specific type of disease and the like. A usual daily dose of oral administration for an adult (body weight: 60 kg) is about 30µg to 10g, preferably 100 µg to 5 g, more preferably 100 µg to 100 mg, and especially preferably 1 mg to 100 mg. A usual daily dose of injection for an adult is about 30 µg to 1 g, preferably 100 µg to 500 mg, more preferably 100 µg to 30 mg, and especially preferably 1 mg to 30 mg. In any case, the daily dose can be administered once or as being divided to several times. Considering that the efficiency is different depending on the administration route, it is expected that the necessary dose may significantly vary. For example, oral administration is considered to require a higher dose than non-oral administration such as intravenous injection. For a child, the dose may be lower than for the adult. The administration method actually used may be significantly vary, and may be depart from the preferable administration method described in this specification. Such a variance of the administration dose level can be appropriately adjusted using a standard and empirical optimizing procedure as well understood in the art.

According to the present invention, the 1,2-dihydropyridine compound represented by general formula (I) can exhibit an excellent dyskinesia suppressing action as a pharmaceutical composition. Hence, the compound represented by general formula (I), a salt thereof, or a solvate thereof is useful as a therapeutic agent for dyskinesia excluding tremor.

Among the symptoms of dyskinesia, "tremor" has been suggested to react well to therapeutic agents for Parkinson's disease, β-blockers and the like (G. Deuschel, P. Krack, Tremors: Differential diagnosis, neurophysiology, and pharmacology. In: Jankovic J and Tolosa E eds., Parkinson's Disease and Movement Disorders. Baltimore: Lippincot Williams & Wilkins, 1998, pp. 419-452) and to accompanied with decreased activity of dopaminergic nerve. According to the present invention, the compound represented by general formula (I), a salt thereof, or a solvate thereof is useful as a therapeutic agent for dyskinesia based on abnormally increased activity of dopaminergic nerve. In other words, the "tremor", among the symptoms of dyskinesia, are not encompassed in the dyskinesia herein.

Herein, the "dyskinesia based on abnormal activity of dopaminergic nerve" means dyskinesia which is improved by a dopamine receptor antagonist or a monoamine depletor, or dyskinesia which is generated by a dopamine receptor agonist or a dopamine metabolism inhibitor.

Hence, according to the present invention, the compound represented by general formula (I), a salt thereof, or a solvate thereof is useful as a therapeutic agent for symptoms including chorea, dystonia, tic, ballismus, athetosis, and myoclonus as dyskinesia based on abnormal activity of dopaminergic nerve, and also as a therapeutic agent for concurrence of these diseases.

In a preferable embodiment of the present invention, the pharmaceutical composition according to the present invention can suppress dyskinesia without causing the symptoms of Parkinson's disease, which would otherwise be caused as a result of activity of dopaminergic nerve being suppressed.

In a more preferable embodiment of the present invention, the pharmaceutical composition according to the present invention can suppress dyskinesia while improving the symptoms of Parkinson's disease caused as a result of activity of dopaminergic nerve being suppressed.

According to the present invention, the compound represented by general formula (I), a salt thereof, or a solvate thereof is useful for the therapy of dyskinesia (excluding tremor) which occurs following neurodegenerative diseases, metabolic diseases or immune diseases, and a drug-induced dyskinesia (excluding tremor), or concurrence thereof.

Herein, dyskinesia which occurs "following" neurodegenerative diseases or the like means dyskinesia (excluding tremor) occurring during the appearance of neurodegenerative diseases or the like appears or dyskinesia (excluding tremor) occurring as one symptom of the neurodegenerative diseases or the like.

Examples of neurodegenerative diseases include Tourette syndrome, spinocerebellar ataxia, cerebral vascular disorder, head injury, and the like.

Examples of metabolic diseases include acanthocytosis, Wilson's disease, glutaric academia, Leigh disease, and the like.

Examples of immune diseases include systemic lupus erythematosus, Sydenham's chorea, chorea gravidarum, and the like.

The drug-induced dyskinesia means dyskinesia (excluding tremor) cause by administration of drugs. The drug-induced dyskinesia encompasses dyskinesia (excluding tremor) induced during or after an administration period of a drug, and dyskinesia (excluding tremor) induced as one action of the administered drug. Examples of the drug-induced dyskinesia (excluding tremor) include dyskinesia (excluding tremor) induced by administration of either or both of a psychotropic agent (e.g., haloperidol, etc.), and a dopamine receptor agonist (e.g., L-DOPA, pramipexole, bromocriptin, lisuride, pergolide, cabergoline, ropinirole, talipexole, etc.), dyskinesia (excluding tremor) induced by administration of a dopamine metabolism inhibitor (e.g., MAO inhibitor, COMT inhibitor, etc.). Examples of the drug-induced dyskinesia (excluding tremor) also include dyskinesia (excluding tremor) induced by combined therapy of a dopamine receptor agonist and a peripheral dopadecarboxylase inhibitor (peripheral levodopadecarboxylase inhibitor) (e.g., carbidopa, benserazide, etc.). Examples of the drug-induced dyskinesia (excluding tremor) also include dyskinesia (excluding tremor) induced by administration of a prodrug of any of the above-mentioned drugs, for example, dyskinesia (excluding tremor) induced by administration of combined use of L-DOPA or a prodrug thereof and a peripheral dopadecarboxylase inhibitor. According to the present invention, the compound represented by general formula (I), a salt thereof, or a solvate thereof is also useful for the therapy of these types of dyskinesia.

Herein, the term "combined use" encompasses a form of administering different compounds concurrently or separately, and a form of administrating a mixture of different compounds concurrently or separately The term "concurrently" means administering at the same timing in one administration schedule, and does not necessary mean administering exactly at the same time. The term "separately" means administering at different timings in one administration schedule.

Herein, examples of the prodrug of the above-mentioned drugs include compounds obtained as a result of, for example, acylation, alklation, phosphorylation, borylation, carbonylation, esterification, amidation, or urethanation of amino group, hydroxyl group, carboxyl group or the like of the above-mentioned drugs; and various other prodrugs. The group of drugs listed above as examples are not comprehensive but are merely representative. Those skilled in the art can prepare various other known prodrugs from the above-mentioned drugs by known methods. For example, one example of a prodrug of dopamine receptor agonist is C₁-C₆ alkylester of L-DOPA, and preferably methylester or ethylester of L-DOPA.

Herein, the term "therapy" means, in general, to obtain a desired pharmacological and/or physiological effect. The effect is preventive in the sense of completely or partially preventing diseases and/or symptoms, and is therapeutic in the sense of partially or completely curing adverse influence caused by diseases and/or symptoms. Herein, the term "therapy" includes an arbitrary therapy for mammalian diseases, especially, human diseases, and includes, for example, the following (a) through (c).
(a) To prevent occurrence of a disease or symptom in a patient who may have diathesis of the disease or symptom but is not diagnosed to have the diathesis.
(b) To inhibit the symptom of a disease, i.e., to block or delay the progress of the disease.
(c) To alleviate the symptom of a disease, i.e., to cause regression of the disease or symptom, or reversal of the progress of the disease or symptom.

The present invention further encompasses use of a 1,2-dihydropyridine compound for producing a therapeutic agent for dyskinesia excluding tremor. In the use according to the present invention, the 1,2-dihydropyridine compound is a compound represented by general formula (I); and is preferably at least one compound selected from 3-(2-cyanophenyl)-5-(2-pyridyl)-1-phenyl-1,2-dihydropyridine-2-on, 3-(2-cyanophenyl)-5-(2-pyridyl)-1-(3-pyridyl)-1,2-dihydropyridine-2-on, 3-(2-fluoropyridine-3-yl)-5-(2-pyridyl)-1-phenyl-1,2-dihydropyridine-2-on, 3-(2-fluoropyridine-3-yl)-5-(2-pyridyl)-1-(3-pyridyl)-1,2-dihydropyridine-2-on, 3-(2-cyanophenyl)-1-phenyl-5-(2-pyrimidinyl)-1,2-dihydropyridine-2-on, 3-(2-cyanophenyl)-1-(3-pyridyl)-5-(2-pyrimidinyl)-1,2-dihydropyridine-2-on, 3-(2-fluoropyridine-3-yl)-1-phenyl-5-(2-pyrimidinyl)-1,2-dihydropyridine-2-on, and 3-(2-cyanopyridine-3-yl)-1-phenyl-5-(2-pyrimidinyl)-I,2-dihydropyridine-2-on, a salt thereof, or a solvate thereof, and is more preferably 3-(2-cyanophenyl)-5-(2-pyridyl)-1-phenyl-1,2-dihydropyridine-2-on, a salt thereof, or a solvate thereof. 3-(2-cyanophenyl)-5-(2-pyridyl)-1-phenyl-1,2-dihydropyridine-2-on is preferably a hydrate.

The present invention encompasses a therapeutic method for dyskinesia, excluding tremor, of administering a 1,2-dihydropyridine compound to a patient. In the method according to the present invention, the 1,2-dihydropyridine compound is a compound represented by general formula (I); and is preferably at least one compound selected from 3-(2-cyanophenyl)-5-(2-pyridyl)-1-phenyl-1,2-dihydropyridine-2-on, 3-(2-cyanophenyl)-5-(2-pyridyl)-1-(3-pyridyl)-1,2-dihydropyridine-2-on, 3-(2-fluoropyridine-3-yl)-5-(2-pyridyl)-1-phenyl-1,2-dihydropyridine-2-on, 3-(2-fluoropyridine-3-yl)-5-(2-pyridyl)-1-(3-pyridyl)-1,2-dihydropyridine-2-on, 3-(2-cyanophenyl)-1-phenyl-5-(2-pyrimidinyl)-1,2-dihydropyridine-2-on, 3-(2-cyanophenyl)-1-(3-pyridyl)-5-(2-pyrimidinyl)-1,2-dihydropyridine-2-on, 3-(2-fluoropyridine-3-yl)-1-phenyl-5-(2-pyrimidinyl)-1,2-dihydropyridine-2-on, and 3-(2-cyanopyridine-3-yl)-1-phenyl-5-(2-pyrimidinyl)-1,2-dihydropyridine-2-on, a salt thereof, or a solvate thereof, and is more preferably 3-(2-cyanophenyl)-5-(2-pyridyl)-1-phenyl-1,2-dihydropyridine-2-on, a salt thereof, or a solvate thereof. 3-(2-cyanophenyl)-5-(2-pyridyl)-1-phenyl-1,2-dihydropyridine-2-on is preferably a hydrate. In the therapeutic method for dyskinesia (excluding tremor) according to the present invention, there is no specific limitation on the administration route or the administration method of the 1,2-dihydropyridine compound, i.e., the compound represented by general formula (I), but the descriptions regarding the above-described pharmaceutical compositions may be referred to for the administration route and the administration method.

Hereinafter, the present invention will be described in more detail by way of examples and production examples, but the present invention is not limited to these examples. The examples and production examples are presented to provide a complete disclosure to those skilled in the art. It is not intended or suggested that the experiments described herein are all or the only experiments which have been performed. Regarding the numerical values (e.g., dose, concentration, etc.), efforts were made in order to guarantee the accuracy, but a certain degree of experimental errors and deviations are considerable, and the numerical values may be altered without departing from the scope of the present invention.

### Examples Evaluation using an L-DOPA induced dyskinesia model

### (1) Production of L-DOPA induced dyskinesia model in monkey

To male crab-eating macaques, MPTP (1-methyl-4-phenyl-1,2,3,6-tetrahydropyridine) was frequently administered intravenously or subcutaneously in a dose of 0.2 to 1 mg/kg to cause Parkinson's disease. To the monkeys, L-3,4-dihydroxyphenylalaninemethylester (L-DOPA methylester) and benserazide, which is a decarboxylase inhibitor were repeatedly administered in a dose at which each monkey exhibited improvement in the symptoms of Parkinson's disease, until dyskinesia expressed. Thus, dyskinesia based on abnormally increased activity of dopaminergic nerve was provoked.

### (2) Evaluation method

In order to check the effect of the substance to be tested, i.e., 3-(2-cyanophenyl)-5-(2-pyridyl)-1-phenyl-1,2-dihydropyridine-2-on, the administration dose of L-DOPA methylester was adjusted to control the severity of the dyskinesia to a certain state.

The behavior before the administration of drugs (L-DOPA methylester and benserazide) was recorded, and then the substance to be tested was administered. 20 minutes after that, L-DOPA methylester and benserazide were administered.

The severity of the symptoms of Parkinson's disease was evaluated based on the items shown in Table 1 in a blinded manner, and shown as the total score.

**Table 1 Indices of the symptoms of Parkinson's disease**

| Range of movement | no movement | head only | head and upper limb | limbs and trunk | walk or stand up |
|---|---|---|---|---|---|
| Score | 4 | 3 | 2 | 1 | 0 |
| Frequency of movement | no movement | rare | often | continuous movement with interval | continuous movement without interval |
| Definition | no movement | a few times/5min | frequent movement | movement > pause | without interval |
| Score | 4 | 3 | 2 | 1 | 0 |
| Speed of movement | no movement | very slow | slow | slightly slow | normal |
| Definition | no movement | extremely slower than normal | obviously slower than normal | slightly slower than normal | Normal speed |
| Score | 4 | 3 | 2 | 1 | 0 |
| Freezing | strong | weak | no freezing | | |

| Definition | no movement or freezing during movement with constrained posture | transient freezing during movement | absent | | |
|---|---|---|---|---|---|
| Score | 2 | 1 | 0 | | |
| posture | crouching | flex | upright | | |
| Definition | flex and face down | flex but face up | upright | | |
| Score | 2 | 1 | 0 | | |
| tremor | sever | mild | No tremor | | |
| Definition | whole body | limited in extremity | absent | | |
| Score | 2 | 1 | 0 | | |

The dyskinesia induced by the side effects of the admistration of L-DOPA methylester and benserazide was evaluated based on the items shown in Table 2 in a blinded manner. The scores of the severity of the dyskinesia in each part of the body and the score of the frequency thereof were added up, and the total score was shown as the dyskinesia score representing the acuity.
Table 2 Indices of the dyskinesia

**Table 2-1 Frequency of dyskinesia**

| Frequency | 0% | Several times/5 min. | Dyskinesia < normal | Dyskinesia > normal | No clear pause of dyskinesia |
|---|---|---|---|---|---|
| Score | 0 | 1 | 2 | 3 | 4 |

**Table 2-2 Definition of acuity of dyskinesia**

| Head | faint | evident | | | |
|---|---|---|---|---|---|
| definition | oral dyskinesia without tongue protration | oral dyskinesia with tongue protration | | | |
| Score | 1 | 2 | | | |
| Trunk | faint | evident | | | |
| definition | rare inconsistent trunk movement with eyes | obvious inconsistent trunk movement with eyes | | | |
| Score | 1 | 2 | | | |
| Upper limb | mild | mild-moderate | moderate | moderate-sever | sever |
| definition | wrist only | wrist and elbow | wrist, elbow and shoulder | interfere normal movement by dyskinesia | only dyskinesic movement |
| Score | 1 | 2 | 3 | 4 | 5 |
| Lower limb | mild | mild-moderate | moderate | moderate-sever | sever |
| definition | only ankle | ankle and knee | ankle, knee and coxa | sway by dyskinesia | tumble down and difficult to stand by dyskinesia |
| Score | 1 | 2 | 3 | 4 | 5 |

The symptoms were evaluated in a time-series manner before the administration of the drugs, and every 30 minutes after the administration of the drugs, for a total of 5 hours. The effect of the substance to be tested was determined as follows. The severity of the dyskinesia and Parkinson's disease occurring to the group of monkeys administered with the drugs and the group of monkeys not administered with the drugs were each scored. After repeated-measures ANOVA, t-test was performed at certain time points. Among the results of the same experiment, the results representing the effects on the dyskinesia are shown in Fig. 1, and the results representing the effects on Parkinson's disease are shown in Fig. 2.

### (3) Results

As a result of this pharmacological experiment, 3-(2-cyanophenyl)-5-(2-pyridyl)-1-phenyl-1,2-dihydropyridine-2-on exhibited the effects which were not realized by any conventional compound; i.e., provided an dyskinesia suppressing action (Fig. 1) without causing the symptoms of Parkinson's disease, which would otherwise be caused as a result of activity of dopaminergic nerve being suppressed and was considered a problem in the conventional therapy of dyskinesia; and in more detail, provided the dyskinesia suppressing action while clearly improving the symptoms of Parkinson's disease (Fig. 2).

### INDUSTRIAL APPLICABILITY

The present invention provides an-excellent therapeutic agent for dyskinesia (excluding tremor). More specifically, an excellent therapeutic agent for dyskinesia (excluding tremor) containing a 1,2-dihydropyridine compound, i.e., a compound represented by general formula (I), preferably, 3-(2-cyanophenyl)-5-(2-pyridyl)-1-phenyl-1,2-dihydropyridine-2-on, a salt thereof, or a solvate thereof is provided, and is usable for the therapy of dyskinesia excluding tremor.

The present invention makes it possible to conduct therapy for dyskinesia without causing the symptoms of Parkinson's disease, which would otherwise be caused as a result of activity of dopaminergic nerve being suppressed and was considered a problem in the conventional therapy of dyskinesia excluding tremor, preferably while clearly improving the symptoms of Parkinson's disease.

## Claims

1. A therapeutic agent for dyskinesia (excluding tremor), comprising a compound represented by the following general formula (I), a salt thereof, or a solvate thereof: 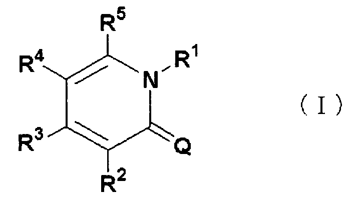 (in the formula,
Q represents =NH, =O or =S;
R¹, R², R³, R⁴ and R⁵ identically or differently represent a group represented by a hydrogen atom, a halogen atom, a C₁-C₆ alkyl group or a formula -X-A;
X represents a single bond, a C₁-C₆ alkylene group which may have a substituent, a C₂-C₆ alkenylene group which may have a substituent, a C₂-C₆ alkynylene group which may have a substituent, -O-, -S-, -CO-, -SO-, -SO₂- -N(R⁶)-, -N(R⁷)-CO-, -CO-N(R⁸)-, -N(R⁹)-CH₂-, -CH₂-N(R¹⁰)-, -CH₂-CO-, -CO-CH₂-, -N(R¹¹)-S(O)ₘ-, -S(O)ₙ-N(R¹²)-, -CH₂-S(O)p-, -S(O)_{q}-CH₂-, -CH₂-O-, -O-CH₂-, -N(R¹³)-CO-N(R¹⁴)-, or -N(R¹⁵)-CS-N(R¹⁶)-;
R⁶, R⁷, R⁸, R⁹, R¹⁰, R¹¹, R¹², R¹³, R¹⁴, R¹⁵ and R¹⁶ identically or differently represent a hydrogen atom, a C₁-C₆ alkyl group or a C₁-C₆ alkoxy group;
m, n, p and q independently represent an integer 0, 1 or 2; and
A represents a C₃-C₈ cycloalkyl group, a C₃-C₈ cycloalkenyl group, a 5- to 14-membered non-aromatic heterocyclic group, a C₆-C₁₄ aromatic hydrocarbon cyclic group, or a 5- to 14-membered aromatic heterocyclic group, each of which may have a substituent;
wherein three groups among R¹, R², R³, R⁴ and R⁵ always identically or differently represent a group represented by -X-A, and the remaining two groups always represent a hydrogen atom, a halogen atom or a C₁-C₆ alkyl group).

2. The therapeutic agent according to claim 1, wherein the dyskinesia is dyskinesia based on abnormally increased activity of dopaminergic nerve.

3. The therapeutic agent according to claim 1, wherein the dyskinesia is at least one selected from the group consisting of chorea, dystonia, tic, ballismus, athetosis, and myoclonus.

4. The therapeutic agent according to claim 1, wherein the dyskinesia is at least one selected from the group consisting of dyskinesia (excluding tremor) which occurs following neurodegenerative diseases, metabolic diseases or immune diseases, and a drug-induced dyskinesia (excluding tremor).

5. The therapeutic agent according to claim 4, wherein the dyskinesia (excluding tremor) which occurs following neurodegenerative diseases is dyskinesia (excluding tremor) which occurs following at least one selected from the group consisting of Tourette syndrome, spinocerebellar ataxia, cerebral vascular disorder, and head injury.

6. The therapeutic agent according to claim 4, wherein the dyskinesia (excluding tremor) which occurs following metabolic diseases is dyskinesia (excluding tremor) which occurs following at least one selected from the group consisting of acanthocytosis, Wilson's disease, glutaric academia, and Leigh disease.

7. The therapeutic agent according to claim 4, wherein the dyskinesia (excluding tremor) which occurs following immune diseases is dyskinesia (excluding tremor) which occurs following at least one selected from the group consisting of systemic lupus erythematosus, Sydenham's chorea, and chorea gravidarum.

8. The therapeutic agent according to claim 4, wherein the drug-induced dyskinesia (excluding tremor) is dyskinesia (excluding tremor) which occurs following administration of a psychotropic agent and/or a dopamine receptor agonist.

9. The therapeutic agent according to claim 4, wherein the drug-induced dyskinesia (excluding tremor) is dyskinesia (excluding tremor) which occurs following administration of a dopamine receptor agonist.

10. The therapeutic agent according to claim 4, wherein the drug-induced dyskinesia (excluding tremor) is dyskinesia (excluding tremor) which occurs following combined use of L-DOPA or a prodrug thereof and a peripheral dopadecarboxylase inhibitor.

11. The therapeutic agent according to claim 1, wherein the compound is at least one selected from the group consisting of 3-(2-cyanophenyl)-5-(2-pyridyl)-1-phenyl-1,2-dihydropyridine-2-on, 3-(2-cyanophenyl)-5-(2-pyridyl)- 1 -(3-pyridyl)- 1,2-dihydropyridine-2-on, 3-(2-fluoropyridine-3-yl)-5-(2-pyridyl)-1-phenyl-1,2-dihydropyridine-2-on, 3-(2-fluoropyridine-3-yl)-5-(2-pyridyl)-1-(3-pyridyl)-1,2-dihydropyridine-2-on, 3-(2-cyanophenyl)-1 -phenyl-5-(2-pyrimidinyl)-1,2-dihydropyridine-2-on, 3-(2-cyanophenyl)- 1 -(3-pyridyl)-5-(2-pyrimidinyl)- 1,2-dihydropyridine-2-on, 3-(2-fluoropyridine-3-yl)-1-phenyl-5-(2-pyrimidinyl)-1,2-dihydropyridine-2-on, and 3-(2-cyanopyridine-3-yl)-1-phenyl-5-(2-pyrimidinyl)-1,2-dihydropyridine-2-on.

12. The therapeutic agent according to claim 1, wherein the compound is 3-(2-cyanophenyl)-5-(2-pyridyl)-1-phenyl-1, 2-dihydropyridine-2-on.

13. The therapeutic agent according to claim 1, wherein the compound is a hydrate 3-(2-cyanophenyl)-5-(2-pyridyl)-1-phenyl-1, 2-dihydropyridine-2-on hydrate.
